# EUROPEAN PATENT APPLICATION

(11) **EP 1 177 797 A1**
(43) Date of publication of application: **06.02.2002**
(21) Application number: 00925617.3
(22) Date of filing: 11.05.2000
(51) Int. Cl.: A61K 45/00, A61K 31/4985, A61K 31/437, A61P 25/22, A61P 25/24, A61P 25/16, C07D 471/04, C07D 487/04

(54) **NOVEL USE**

(30) Priority: 12.05.1999 JP 13110899
(71) Applicant: FUJISAWA PHARMACEUTICAL CO., LTD., Osaka-shi Osaka 541-8514 (JP)
(72) Inventor: MATSUOKA, Nobuya, Souraku-gun, Kyoto 619-0237 (JP); MORIGUCHI, Akira, Ibaraki-shi, Osaka 567-0046 (JP); TADA, Miho, Amagasaki-shi, Hyogo 661-0002 (JP); MIHARA, Takuma, Ikoma-gun, Nara 636-0938 (JP)
(74) Representative: Gille Hrabal Struck Neidlein Prop Roos
(86) International application number: JP0003015
(87) International publication number: WO0069464

(57) **Abstract**

Preventives and/or remedies for Parkinson's disease and symptoms associating therewith such as anxiety, depression and/or memory disorder which contain as the active ingredient an adenosine A₁A₂ₐ receptor dual antagonist or salts thereof.

## Description

### TECHNICAL FIELD

This invention relates to a pharmaceutical composition for the prevention and/or treatment of Parkinson's disease and concomitant symptoms thereof such as anxiety, depression and/or memory impairment, among others comprising an adenosine A₁A₂ₐ-receptor dual antagonist or a salt thereof as an active ingredient and so is useful in the pharmaceutical field.

### BACKGROUND ART

Based inter alia on the investigations made in model animals, the potential utility of an adenosine A₂ₐ-receptor antagonist as a therapeutic agent for motor neuron diseases such as Parkinson's disease has been recently reported. Meanwhile, it is reported that an adenosine A₁-receptor antagonist shows learning memory improving effects in several animal models.

It is, therefore, suggested that a compound having both adenosine A₁-receptor antagonizing and adenosine A₂ₐ-receptor antagonizing activities in one may be a new kind of therapeutic drug for Parkinson's disease, which has a battery of antidementia, anxiolytic, antidepression and other actions. Patients with Parkinson's disease present with certain mental symptoms such as depression and dementia but the currently available therapeutic drugs for Parkinson's disease are not effective in controlling those accessory symptoms. In contrast, an adenosine A₁A₂ₐ-receptor dual antagonist is not only considered to cure the cardinal morbidity of Parkinson's disease but also expected to palliate its accessory mental symptoms.

However, it is known that a compound having an adenosine antagonist activity possesses various physiological actions in addition to the above-mentioned, and it is not clear whether an intentional pharmacological action is exhibited or not, even if an adenosine A₁A₂ₐ-receptor dual antagonist is used. Moreover, it is unknown at all what measure of a strength of an action etc. of an adenosine A₁A₂ₐ-receptor dual antagonist is need in order to treat an aimed symptom effectively.

The inventors of the present invention succeeded in solving these problems by the exertive study for the above point.

### DISCLOSURE OF INVENTION

The inventors of this invention found that a compound having adenosine A₁-receptor and adenosine A₂ₐ-receptor antagonizing activities is effective in preventing and/or treating Parkinson's disease and concomitant symptoms thereof such as anxiety, depression and/or memory impairment, among others, and have developed this instant invention. This invention, therefore, accomplishes the above object by providing a composition for the prevention and/or therapy of Parkinson's disease and concomitant symptoms thereof such as anxiety, depression and/or memory impairment, among others which comprises an adenosine A₁A₂ₐ-receptor dual antagonist or a salt thereof as an active ingredient.

### BEST MODE FOR CARRYING OUT THE INVENTION

This invention is carried into practice by administering an adenosine A₁A₂ₐ-receptor dual antagonist or a salt thereof or a pharmaceutical composition comprising an adenosine A₁A₂ₐ-receptor dual antagonist or a salt thereof as an active ingredient to a patient with Parkinson's disease and concomitant symptoms thereof such as anxiety, depressive disorder and/or memory impairment, among others.

The term "adenosine A₁A₂ₐ-receptor dual antagonist" is used herein to mean a substance which antagonizes both the adenosine A₁ receptor and the adenosine A₂ₐ receptor.

The existence and intensity of "adenosine A₁-receptor antagonizing activity" and/or "adenosine A₂ₐ-receptor antagonizing activity" can be ascertained and assessed typically by the following test method. The intensity of adenosine A₁-receptor antagonizing action and of adenosine A₂ₐ-receptor antagonizing action as referred to in the appended claims also means the value found by the method described below.

### "Estimation of Adenosine A₁ and A₂ₐ-receptor antagonizing activity"

### Method

Membrane fractions prepared from the CHO cells which transfected stably with human recombinant A₁ or A₂ₐ receptors were incubated for 1 hr at 25°C with test compounds, ADA, 50 mM Tris buffer and [3H]-DPCPX (final 4 nM) or [3H]-CGS21680 (final 15 nM), respectively. Reaction mixtures were filtrated with 96-well harvestor to separate free ligands from bound fraction using GF/C filter. Radioactivity of the dried filter was counted, and specific binding of each labelled rigands was calculated.

For example, when (A) 3-[2-(Thiazol-2-ylmethyl)-3-oxo-2,3-dihydropyridazin-6-yl]-2-phenylpyrazolo[1 ,5-a]pyridine is used as a test compound, the result is as follows.

The inhibition rates (%) for both of Adenosine A₁ and A₂ₐ-receptor were not less than 80% at a concentration of 1.0×10⁻⁶ (M). The affinity for the adenosine A₁-receptor is 36 times as high as its affinity for the adenosine A₂ₐ receptor.

In carrying this invention into practice, it is preferable to use an adenosine A₁A₂ₐ-receptor dual antagonist having sufficiently high adenosine A₂ₐ-receptor antagonizing activity. For example, generally a compound giving an IC₅₀ value of not more than 100 nM as determined by the above test method is preferably used and one with said IC value of not more than 50 nM is more preferably used. [In selecting the antagonist, one skilled in the art should take experimental errors into consideration.] However, the above is not an exclusive choice but one skilled in the art may judiciously select the optimum adenosine A₁A₂ₐ-receptor dual antagonist in carrying the invention into practice.

Incidentally, even a substance having some additional pharmacologic activity other than adenosine A₁A₂ₐ-receptor dual antagonizing activity can also be used in this invention. Moreover, the adenosine A₁A₂ₐ-receptor dual antagonist for use in this invention is a substance whose affinity for the adenosine A₁-receptor is preferably 0.25~40 times, more preferably 8~40 times, as high as its affinity for the adenosine A₂ₐ receptor.

The adenosine A₁A₂ₐ-receptor dual antagonists for use in this invention are not exclusive but many kinds of compounds may be included.

For example, compounds reported to have antagonizing activity against the adenosine A₁-receptor or the adenosine A₂ₐ receptor may include adenine derivative, barbiturate derivative, benzimidazole derivative, benzo[1,2-c:5,4-c']dipyrazole derivative, benzo[b]furan derivative, benzo[g]pteridine-2,4-dione derivative, β -carboline derivative, dibenz[b,f]azepine derivative, flavone derivative, imidazo[1,2-a]pyrazine derivative, imidazo[4,5-b]pyridine derivative, imidazo[4,5-c]quinoline derivative, imidazo[4,5-e][1,4]diazepine-5,8-dione derivative, imidazo[4,5-f]quinazoline-7,9-dione derivative, imidazo[4,5-g]quinazoline-6,8-dione derivative, imidazo[1,2-a]quinoxaline derivative, imidazoline derivative, imidazotriazolopyrimidine derivative, pteridine-2,4-dione derivative, pyrazole derivative, pyrazolo[1,5-a]pyradine derivative, pyrazolo[1,5-a]pyridine derivative, pyrazolo[3,4-b]pyridine derivative, pyrazolo[3,4-d]pyrimidine derivative, pyrazolo[4,3-d]pyrimidine derivative, pyrazolo[4,3-c]quinoline derivative, pyrimidine derivative, pyrimido[4,5-b] (tetrahydro)indole derivative derivative, pyrrolo[2,3-d]pyrimidine derivative, quinazoline derivative, quinoline derivative, thiazolo[3,2-a]pyrimidine derivative, thiazolo[2,3-b]quinazoline derivative, thiazolo[4,5-b]pyrimidine-5,7-dione derivative, thiazolo[5,4-d]pyrimidine-5,7-dione derivative, thiophene derivative, triazolo[3,2-a] [2,7]naphthyridine derivative, triazolopurine derivative, [1,2,4]triazolo[4,3-b]pyridazine derivative, triazolo[1,5-a]pyrimidine derivative, triazolo[1,5-c]pyrimidine derivative, [1,2,4]triazolo[1,5-c]quinazoline derivative, [1,2,4]triazolo[4,3-a]quinoxaline derivative, triazolo[1,5-a]triazine derivative, xanthine derivative, mesoionic xanthine derivative, and the like.

The above compounds can be illustrated as follows.
1)adenine derivative described in WO99/35147, WO99/38532 and WO96/06845, etc,
2)barbiturate derivative described in Naunyn Schmiedeberg's Arch. Pharmacol. 1987, 336, 211-217,
3)benzimidazole derivative described in JP10-182636,
4)benzo[1,2-c:5,4-c']dipyrazole derivative described in J. Med. Chem. 1988, 31, 2034-2039,
5)benzo[b]furan derivative described in Tetrahedron Lett. 1991, 32, 2061-2064,
6)benzo[g]pteridine-2,4-dione derivative described in Biochem. Pharmacol. 1981, 30, 325-333,
7) β-carboline derivative described in Biochem. Pharmacol. 1988, 37, 655-664,
8)dibenz[b,f]azepine derivative described in Eur. J. Pharmacol. 1982, 82, 195-197,
9)flavone derivative described in WO97/27177,
10) imidazo[1,2-a]pyrazine derivative described in Biochem. Pharmacol. 1988, 37, 655-664,
11) imidazo[4,5-b]pyridine derivative described in Biochem. Pharmacol. 1988, 37, 655-664,
12) imidazo[4,5-c]quinoline derivative described in J. Med. Chem. 1991, 34, 1202-1206,
13) imidazo[4,5-e][1,4]diazepine-5,8-dione derivative described in J. Med. Chem. 1990, 33, 2818-2821,
14) imidazo[4,5-f]quinazoline-7,9-dione derivative described in *J. Med*. Chem. 1989, 32, 2247-2254,
15) imidazo[4,5-g]quinazoline-6,8-dione derivative described in J. Med. Chem. 1989, 32, 2247-2254,
16) imidazo[1,2-a]quinoxaline derivative described in WO97/19079,
17) imidazoline derivative described in Biochem. Pharmacol. 1988, 37, 655-664,
18) imidazotriazolopyrimidine derivative described in WO99/65912, WO00/12511, etc,
19) pteridine-2,4-dione derivative described in Biochem. Pharmacol. 1981, 30, 325-333,
20) pyrazole derivative described in WO97/01551, WO99/24424, etc,
21) pyrazolo[1,5-alpyridine derivative described in JP64-45385, JP2-243689, JP4-253978, JP5-112566, WO95/18128, WO98/03507, etc,
22) pyrazolo[3,4-b]pyridine derivative described in Biochem. Pharmacol. 1982, 31, 1441-1442,
23) pyrazolo[3,4-d]pyrimidine derivative described in WO99/67243,
24) pyrazolo[4,3-d]pyrimidine derivative described in J. Med. Chem. 1987, 30, 91-96,
25) pyrazolo[4,3-c]quinoline derivative described in Life Sci. 1982, 30, 363-372,
26) pyrimidine derivative described in WO99/11633,
27) pyrimido[4,5-b](tetrahydro)indole derivative described in J. Med. Chem. 1990, 33, 2822-2828,
28) pyrrolo[2,3-d]pyrimidine derivative described in WO99/62518,
29) quinazoline derivative described in Physiology and Pharmacology; Paton,D.M.,Ed.; Taylor & Francia: London, 1988; pp39-49,
30) quinoline derivative described in WO99/26627,
31) thiazolo[3,2-a]pyrimidine derivative described in WO97/33879,
32) thiazolo[2,3-b]quinazoline derivative described in Physiology and Pharmacology; Paton, D.M., Ed.; Taylor & Francia: London, 1988; pp39-49,
33) thiazolo[4,5-b]pyrimidine-5,7-dione derivative described in Biochem. Pharmacol. 1988, 37, 655-664,
34) thiazolo[5,4-d]pyrimidine-5,7-dione derivative described in Life Sci. 1984, 34, 2117-2128,
35) thiophene derivative described in WO99/21617,
36) triazolo[3,2-a][2,7]naphthyridine derivative described in USP5780481,
37) triazolopurine derivative described in WO98/03511,
38) [1,2,4]triazolo[4,3-b]pyridazine derivative described in Biochem. Pharmacol. 1988, 37, 655-664, 39) triazolo[1,5-a]pyrimidine derivative described in WO99/43678,
40) triazolo[1,5-c]pyrimidine derivative described in WO98/42711,
41) [1,2,4]triazolo[1,5-c]quinazoline derivative described in Biochem. Pharmacol. 1988, 37, 655-664,
42) [1,2,4]triazolo[4,3-a]quinoxaline derivative described in Biochem. Pharmacol. 1988, 37, 655-664,
43) triazolo[1,5-a]triazine derivative described in WO95/03806, WO94/14812, EP0459702, etc,
44) xanthine derivative described in EP0386675, EP0415456, JP2-247180, WO90/12797, WO97/04782, WO99/12546, WO99/54331, WO98/22465, etc,
45) mesoionic xanthine derivative described in Biochem. Pharmacol. 1988, 37, 655-664
In the above compounds and salts thereof by the method described above "Estimation of Adenosine A₁ and A₂ₐ-receptor antagonizing activity", the intensity of adenosine A₁-receptor antagonizing activity and/or adenosine A₂ₐ-receptor antagonizing activity can be studied and elected compounds having preferable profile, and can be used as adenosine A₁A₂ₐ-receptor dual antagonist of the present invention.

Defining concretely said adenosine A₁A₂ₐ-receptor dual antagonist in structural terms, it may for example be a compound as follows.
1. A compound selected from among pyrazolopyridine compounds of the following general formula (I) or salts thereof: 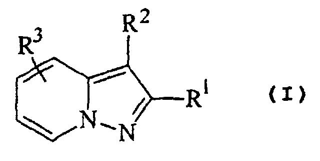 [wherein R¹ is lower alkyl, aryl optionally having one or more suitable substituent(s), or a heterocyclic group;
   R² is a group of the formula: 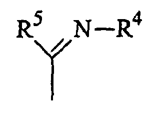 (where R⁴ is protected amino or hydroxyl and R⁵ is hydrogen or lower alkyl);
   cyano;
   a group of the formula:

      -A-R⁶

      (wherein R⁶ is acyl, and A is lower aliphatic hydrocarbon group optionally having one or more suitable
   substituent(s)) ;
   amidated carboxyl group;
   unsaturated heterocyclic group optionally having one or more suitable substituent(s);
   amino; or
   protected amino; and
   R³ is hydrogen, lower alkyl, lower alkoxy, or halogen]

   Hereat, the selection of a compound is carried out by judiciously selection of a suitable compound having preferred profile after estimating strength of adenosine A₁ and A₂ₐ-receptor antagonizing activity by the above method of "Estimation of Adenosine A₁ and A₂ₐ-receptor antagonizing activity"
   The above pyrazolopyridine compound (I) includes but is not limited to the known compounds described in JP Kokai S64-45385, JP Kokai H2-243689, JP Kokai H4-253978, JP Kokai H5-112566, WO 95/18128 and WO 98/03507.
   Suitable salts of said pyrazolopyridine compound (I) are pharmaceutically acceptable salts of the conventional type and, as such, include metal salts, for example alkali metal salts such as sodium salt, potassium salt, etc. and alkaline earth metal salts such as calcium salt, magnesium salt, etc.; ammonium salt; salts with organic bases, such as trimethylamine salt, triethylamine salt, pyridine salt, picoline salt, dicyclohexylamine salt, N,N'-dibenzylethylenediamine salt, etc.; salts with organic acids, such as acetate, trifluoroacetate, maleate, tartrate, fumarate, methanesulfonate, benzenesulfonate, formate, toluenesulfonate, etc.; salts with inorganic acids, such as hydrochloride, hydrobromide, hydroiodide, sulfate, phosphate, etc.; and salts with amino acids such as arginine, aspartic acid, glutamic acid, and so on.
   Suitable examples and detailed remarks or comments on the various definitions for said pyrazolopyridine compound (I) are as follows.
   The term "lower" means 1 to 6 carbon atoms unless otherwise indicated.
   The term "higher" means 7 to 20 carbon atoms unless otherwise indicated.
   Suitable "lower aliphatic hydrocarbon group" may include the lower alkyl, lower alkenyl and lower alkynyl defined below.
   Suitable "lower alkyl group" may include straight-chain or branched-chain alkyl groups such as methyl, ethyl, propyl, isopropyl, butyl, tert-butyl, pentyl, hexyl, etc.; among these, (C₁-C₄) alkyl groups are preferred and methyl, ethyl, propyl and isopropyl are more preferred.
   Suitable "lower alkenyl group" may include straight-chain or branched-chain alkenyl groups such as vinyl, 1-methylvinyl, 2-methylvinyl, 1-propenyl, 2-propenyl, 1-butenyl, 2-methyl-1-propenyl, 1,3-butadienyl, 1-pentenyl, 4-pentenyl, 1-hexenyl, 1,4-hexadienyl, 5-hexenyl, etc.; among these, (C₂-C₄) alkenyl groups are preferred and vinyl, 1-methylvinyl, 2-methylvinyl and 1,3-butadienyl are more preferred.
   Suitable "lower alkynyl group" may include straight-chain or branched-chain alkynyl groups such as ethynyl, 1-propynyl, 1-methylethynyl, 2-butynyl, 2-methyl-3-butynyl, 2-pentynyl, 1-hexynyl, etc.; among these, (C₂-C₄) alkynyl groups are preferred and ethynyl is more preferred.
   The "lower aliphatic hydrocarbon group" mentioned above may have one or more, preferably 1-3, suitable substituent(s) such as halogen, e.g. chloro, bromo, fluoro and iodo.
   Suitable "protected amino group" may include lower alkylamino groups such as methylamino, ethylamino, propylamino, butylamino, tert-butylamino,pentylamino, hexylamino, etc.; di (lower) alkylamino groups such as dimethylamino, diethylamino, N-ethylpropylamino, dibutylamino, N-(tert-butyl)pentylamino, dihexylamino, etc.; and amino groups substituted by the conventional amino-protecting groups, for example the "acylamino group" defined below.
   Suitable "acylamino group" may include ureido; lower alkanoylamino groups, such as formylamino, acetylamino, propionylamino, butyrylamino, isobutyrylamino, pivaloylamino, hexanoylamino, etc.; lower alkoxycarbonylamino groups such as methoxy-carbonylamino, ethoxycarbonylamino, propoxy-carbonylamino, tert-butoxycarbonylamino, pentyloxy-carbonylamino, hexyloxycarbonylamino, etc.; lower alkoxycarbonyl(lower)alkanoylamino groups, such as methoxycarbonylacetylamino, ethoxycarbonyl-acetylamino, 2-(propoxycarbonyl)propionylamino, 4-(tert-butoxycarbonyl)butyrylamino, 2-(butoxy-carbonylmethyl)propionylamino, 2-methyl-2-(pentyloxycarbonylmethyl)propionylamino, 6-hexyloxycarbonylhexanoylamino, etc.; and lower alkanesulfonylamino groups, such as methanesulfonylamino, ethanesulfonylamino, propanesulfonylamino, butanesulfonylamino, tert-butanesulfonylamino, pentanesulfonylamino, hexanesulfonylamino, and so on.
   The "lower alkanoylamino group" mentioned above may have suitable substituents such as di(lower)alkylamino group, e.g. dimethylamino, N-methyl-N-ethylamino, dipropylamino, di-tert-butylamino, N-pentyl-N-hexylamino, etc. or cycloamino group optionally substituted by lower alkyl, e.g. piperidino etc. As the preferred examples of the "lower alkanoylamino group having suitable substituents", there can be mentioned lower alkanoylamino groups having di(lower)alkylamino, such as dimethylaminocarbonylamino, 2-dimethylamino-acetylamino, 2-(N-methyl-N-ethylamino) acetylamino, 2-dimethylaminopropionylamino, 3-dipropylamino-butyrylamino, 2-(di-tert-butylamino)-2-methyl-propionylamino, 2-dimethylaminomethyl-2-methylpropionylamino, 6-(N-pentyl-N-hexylamino)-hexanoylamino, etc.; and lower alkanoylamino groups having a cycloamino group which, in turn, may be substituted by lower alkyl, such as piperidino-carbonylamino, 2-piperidinoacetylamino, 2-(2-methylpiperidino)acetylamino, 2-(2-ethyl-piperidino)acetylamino, 2-piperidinopropionylamino, 3-(2-ethylpiperidino)butyrylamino, 2-(4-ethyl-piperidino)-2-methylpropionylamino, 2-piperidinomethyl-2-methylpropionylamino, 6-(3-propylpiperidino)hexanoylamino, and so on.
   The preferred species of said "acylamino group" includes ureido, (C₁-C₄)alkanoylamino, (C₁-C₄)alkoxy-carbonyl(C₁-C₄)alkanoylamino, di(C₁-C₄)alkylamino(C₁-C₄)alkanoylamino, (C₁-C₄)alkylpiperidino(C₁-C₄)alkanoylamino, (C₁-C₄)alkoxycarbonylamino, (C₁-C₄)alkanesulfonylamino, (C₁-C₄)alkylamino and di(C₁-C₄)alkylamino. Among these, the still more preferred are ureido, acetylamino, 2-(ethoxycarbonyl)-acetylamino, 2-dimethylaminoacetylamino, 2-(2-ethylpiperidino)acetylamino, methoxycarbonylamino, methanesulfonylamino, methylamino and dimethylamino.
   Suitable "acyl group" may include lower alkanoyl groups such as formyl, acetyl, propionyl, butyryl, isobutyryl, pivaloyl, hexanoyl, etc.; carboxy; and protected carboxy.
   As the preferred examples of said "protected carboxy", these can be mentioned esterified carboxyl groups, the preferred examples of which are lower alkoxycarbonyl groups such as methoxycarbonyl, ethoxycarbonyl, propoxycarbonyl, butoxycarbonyl, tert-butoxycarbonyl, pentyloxycarbonyl, hexyloxycarbonyl, etc., which may optionally have a nitrogen-containing heterocyclic group;
   N-(lower)alkylcarbamoyl groups such as N-methylcarbamoyl, N-ethylcarbamoyl, N-isopropyl-carbamoyl, N-butylcarbamoyl, N-pentylcarbamoyl, N-hexylcarbamoyl, etc.;
   N-(higher)alkylcarbamoyl groups such as N-heptylcarbamoyl, N-(2-methylheptyl)carbamoyl, N-nonylcarbamoyl, N-decanylcarbamoyl, N-tricyclo-[3.3.1.1.^{3,7}]decanylcarbamoyl, N-undecanylcarbamoyl, N-(bicyclo[4.3.2]undecanyl)carbamoyl, N-dodecanylcarbamoyl, N-tridecanylcarbamoyl, N-tetradecanylcarbamoyl, N-pentadecanylcarbamoyl, N-hexadecanylcarbamoyl, N-heptadecanylcarbamoyl, N-octadecanylcarbamoyl, N-nonadecanylcarbamoyl, N-eicosanylcarbamoyl, etc.;
   N,N-di(lower)alkylcarbamoyl groups such as N,N-dimethylcarbamoyl, N,N-diethylcarbamoyl, N-methyl-N-ethylcarbamoyl, N,N-dipropylcarbamoyl, N,N-di(tert-butyl)carbamoyl, N-pentyl-N-hexylcarbamoyl, etc.;
   N-(lower)alkyl-N-ar(lower)alkylcarbamoyl groups such as N-methyl-N-benzylcarbamoyl etc.; and
   amidated carboxy groups, the preferred examples of which are groups of the formula:

      -CO-R_{N}

      (wherein R_{N} is nitrogen-containing heterocyclic group optionally having one or more suitable substituents; said nitrogen-containing heterocyclic group may contain other hetero atoms such as N, O and S).

   Suitable "nitrogen-containing heterocyclic group" may include saturated or unsaturated monocyclic or polycyclic heterocyclic groups, for example:
   unsaturated 3-8-membered (more preferably 5-7-membered) monocyclic heterocyclic groups containing 1~4 nitrogen atoms, such as azepinyl (e.g. 1H-azepinyl), pyrrolyl, pyrrolinyl, imidazolyl, pyrazolyl, pyridyl and its N-oxide, dihydropyridyl, pyrimidinyl, pyrazinyl, pyridazinyl, triazolyl (e.g. 4H-1,2,4-triazolyl, 1H-1,2,3-triazolyl, 2H-1,2,3-triazolyl), and tetrazolyl (e.g. 1H-tetrazolyl, 2H-tetrazolyl);
   saturated 3~8-membered (more preferably 5~7-membered) monocyclic heterocyclic groups containing 1-4 nitrogen atoms, such as perhydroazepinyl (e.g. perhydro-1H-azepinyl), pyrrolidinyl, imidazolidinyl, piperidino, piperazinyl, etc.;
   unsaturated fused heterocyclic groups containing 1~4 nitrogen atoms, such as indolyl, isoindolyl, indolizinyl, benzimidazolyl, quinolyl, isoquinolyl, indazolyl, benzotriazolyl, etc.;
   saturated fused heterocyclic groups containing 1~4 nitrogen atoms, such as 7-azabicyclo[2.2.1]heptyl, 3-azabicyclo[3.2.2]nonyl, etc.;
   unsaturated 3-8-membered (more preferably 5- or 6-membered) monocyclic heterocyclic groups containing 1 or 2 oxygen atoms and 1-3 nitrogen atoms, such as oxazolyl, isoxazolyl, and oxadiazolyl (e.g. 1,2,4-oxadiazolyl, 1,3,4-oxadiazolyl, 1,2,5-oxadiazolyl);
   saturated 3~8-membered (more preferably 5- or 6-membered) monocyclic heterocyclic groups containing 1 or 2 oxygen atoms and 1~3 nitrogen atoms, such as morpholinyl, sydnonyl, etc.;
   unsaturated fused heterocyclic groups containing 1 or 2 oxygen atoms and 1~3 nitrogen atoms, such as benzoxazolyl, benzoxadiazolyl, etc.;
   unsaturated 3-8-membered (more preferably 5- or 6-membered) monocyclic heterocyclic groups containing 1 or 2 sulfur atoms and 1~3 nitrogen atoms, such as thiazolyl, isothiazolyl, thiadiazolyl (e.g. 1,2,3-thiadiazolyl, 1,2,4-thiadiazolyl, 1,3,4-thiadiazolyl, 1,2,5-thiadiazolyl), dihydrothiazinyl, etc.;
   saturated 3~8-membered (more preferably 5- or 6-membered) monocyclic heterocyclic groups containing 1 or 2 sulfur atoms and 1~3 nitrogen atoms, such as thiazolidinyl etc.; and
   unsaturated fused heterocyclic groups containing 1 or 2 sulfur atoms and 1-3 nitrogen atoms, such as benzothiazolyl, benzothiadiazolyl, and so on.

   The preferred, among the groups mentioned just above, are saturated 3-8-membered monocyclic heterocyclic groups containing 1~4 nitrogen atoms, saturated fused heterocyclic groups containing 1~4 nitrogen atoms, and saturated 3-8-membered monocyclic heterocyclic groups containing 1 or 2 oxygen atoms and 1~3 nitrogen atoms.
   The "nitrogen-containing heterocyclic group" mentioned above may have one or more suitable substituents, for example said lower alkyl groups, hydroxy(lower)alkyl groups such as hydroxymethyl, 1-hydroxyethyl, 2-hydroxyethyl, 3-hydroxypropyl, 2-hydroxybutyl, 1-methyl-1-hydroxymethylethyl, 4-hydroxypentyl, 3-hydroxyhexyl, etc.; lower alkoxy(lower)alkyl groups such as methoxymethyl, 2-methoxyethyl, 1-ethoxyethyl, 3-propoxypropyl, 2-(tert-butoxy)butyl, 5-pentyloxypentyl, 3-hexyloxyhexyl, etc.; acyloxy(lower)alkyl groups such as lower alkanoyloxy(lower)alkyl, e.g. acetoxymethyl, 1-acetoxyethyl, 2-acetoxyethyl, 2-propionyloxyethyl, 3-propionyloxypropyl, 2-butyryloxybutyl, 4-pivaloyloxypentyl, 6-hexanoyloxyhexyl, etc.; protected carboxy such as said lower alkoxycarbonyl groups; carboxy; and acyl(lower)alkyl groups such as lower alkanoyl(lower)alkyl groups (e.g. formylmethyl, 1-formylethyl, 2-acetylethyl, 2-formylpropyl, 3-propionylpropyl, 4-formylbutyl, 2-butyrylbutyl, 1-(formylmethyl)ethyl, 3-formylpentyl, 1-isobutyrylpentyl, 4-pivaloylpentyl, 2-formylhexyl, 6-hexanoylhexyl, etc.), carboxy(lower)alkyl groups (e.g. carboxymethyl, 1-carboxyethyl, 2-carboxypropyl, 1-(carboxymethyl)ethyl, 4-carboxybutyl, 3-carboxypentyl, 2-carboxyhexyl, etc.), protected carboxy(lower)alkyl groups [The preferred are esterified carboxy(lower) alkyl groups and the still more preferred are lower alkoxycarbonyl(lower)alkyl groups such as methoxycarbonylmethyl, 2-methoxycarbonylethyl, 1-ethoxycarbonylethyl, 2-propoxycarbonylpropyl, 1-(methoxycarbonylmethyl)ethyl, 4-t-butoxycarbonyl-butyl, 3-pentyloxycarbonylpentyl, 2-hexyloxy-carbonylhexyl, etc.; and amidated carboxy(lower)alkyl groups, more preferably carbamoyl(lower)alkyl, N- (lower)alkylcarbamoyl (lower)alkyl (e.g. N-ethylcarbamoylmethyl), N,N-di(lower)alkylcarbamoyl(lower)alkyl (e.g. N,N-diethylcarbamoylmethyl); etc.], among others.
   The preferred examples of said "nitrogen-containing heterocyclic group optionally having one or more suitable substituents" include piperidino optionally having 1~4 suitable substituents selected from the class consisting of (C₁-C₄)alkyl, hydroxy(C₁-C₄)alkyl, (C₁-C₄)alkoxy(C₁-C₄)alkyl, (C₁-C₄)alkanoyloxy(C₁-C₄)alkyl, (C₁-C₄)alkoxycarbonyl, carboxy, (C₁-C₄)alkanoyl(C₁-C₄)alkyl, carboxy(C₁-C₄)alkyl, (C₁-C₄)alkoxycarbonyl(C₁-C₄)alkyl, carbamoyl(C₁-C₄)alkyl, N-(C₁-C₄)alkylcarbamoyl(C₁-C₄)alkyl and N,N-di(C₁-C₄)alkylcarbamoyl(C₁-C₄)alkyl, such as piperidino, 2-methylpiperidino, 2-ethylpiperidino, 3-ethylpiperidino, 4-ethylpiperidino, 2-propylpiperidino, 4-isopropylpiperidino, 2-butylpiperidino, 3-(tert-butyl)piperidino, 2,2,6,6-tetramethylpiperidino, 2,2-dimethyl-6,6-diethylpiperidino, 2-hydroxymethylpiperidino, 3-hydroxymethylpiperidino, 2-(1-hydroxyethyl)-piperidino, 2-(2-hydroxyethyl)piperidino, 3-(2-hydroxyethyl)piperidino, 4-(2-hydroxyethyl)-piperidino, 2-(3-hydroxypropyl)piperidino, 3-(2-hydroxybutyl)piperidino, 2-(1-methyl-1-hydroxy-methylethyl)piperidino, 2-methoxymethylpiperidino, 2- (2-methoxyethyl) piperidino, 2- (1-ethoxyethyl)-piperidino, 3-(3-propoxypropyl)piperidino, 4-{2-(tert-butoxy)butyl}piperidino, 2-acetoxy-methylpiperidino, 3-(1-acetoxyethyl)piperidino, 2-(2-acetoxyethyl)piperidino, 3- (2-propionyl-oxyethyl)piperidino, 4-(3-propionyloxypropyl)-piperidino, 2-(2-butyryloxybutyl)piperidino, 2-methoxycarbonylpiperidino, 2-ethoxycarbony-lpiperidino, 2-propoxycarbonylpiperidino, 3-butoxycarbonylpiperidino, 4-(tert-butoxycarbonyl)-piperidino, 2-carboxypiperidino, 3-carboxypiperidino, 4-carboxypiperidino, 2-(2-hydroxyethyl)-3-methyl-piperidino, 2-(2-hydroxyethyl)-4-carboxypiperidino, 2-formylmethylpiperidino, 2-(1-formylethyl)piperidino, 3-(2-acetylethyl)-piperidino, 4-(2-formylpropyl)piperidino, 2-(3-propionylpropyl)piperidino, 2-(4-formylbutyl)-piperidino, 3-(2-butyrylbutyl)piperidino, 2-[1-(formylmethyl)ethyl]piperidino, 2-carboxy-methylpiperidino, 2-(1-carboxyethyl)piperidino, 3-(2-carboxypropyl)piperidino, 4-[1-(carboxymethyl)-ethyl]piperidino, 2-(4-carboxybutyl)piperidino, 2-methoxycarbonylmethylpiperidino, 2-(2-methoxy-carbonylethyl)piperidino, 3-(1-ethoxycarbonyl-ethyl)piperidino, 4-(2-propoxycarbonylpropyl)-piperidino, 2-[1-(methoxycarbonylmethyl)]ethyl]-piperidino, 2-(4-t-butoxycarbonylbutyl)piperidino, etc;
   pyrrolidin-1-yl which may be substituted by (C₁-C₄)alkoxy(C₁-C₄)alkyl, such as pyrrolidin-1-yl, 2-methoxymethylpyrrolidin-1-yl, 2-(2-methoxyethyl)-pyrrolidin-1-yl, 2-(1-ethoxyethyl)pyrrolidin-1-yl, 3-(3-propoxypropyl)pyrrolidin-1-yl, 3-{2-(tert-butoxy)butyl}pyrrolidin-1-yl, etc.;
   perhydroazepin-1-yl, such as perhydro-1H-azepin-1-yl;
   piperazin-1-yl which may be substituted by (C₁-C₄)alkyl, such as piperazin-1-yl, 2-methylpiperazin-1-yl, 3-methylpiperazin-1-yl, 4-methylpiperazin-1-yl, 2-ethylpiperazin-1-yl, 3-propylpiperazin-1-yl, 4-isopropylpiperazin-1-yl, 2-butylpiperazin-1-yl, 3-(tert-butyl)piperazin-1-yl, etc.;
   morpholino;
   7-azabicyclo[2.2.1]heptan-7-yl; and
   3-azabicyclo[3.2.2]nonan-3-yl; among others.
      The most preferred are piperidino, 2-methylpiperidino, 2-ethylpiperidino, 3-ethylpiperidino, 4-ethyl-piperidino, 2-propylpiperidino, 2,2,6,6-tetramethyl-piperidino, 2-hydroxymethylpiperidino, 2-(2-hydroxy-ethyl)piperidino, 4-(2-hydroxyethyl)piperidino, 2-methoxymethylpiperidino, 2-(2-methoxyethyl)-piperidino, 2-acetoxymethylpiperidino, 2-(2-acetoxyethyl)piperidino, 2-ethoxycarbonylpiperidino, 2-carboxypiperidino, 2-(methoxycarbonylmethyl)-piperidino, 2-carboxymethylpiperidino, 2-carbamoyl-methylpiperidino, 2-(N-ethylcarbamoylmethyl)-piperidino, 2-N,N-diethylcarbamoylmethyl)piperidino, pyrrolidin-1-yl, 2-methoxymethylpyrrolidin-1-yl, perhydro-lH-azepin-1-yl, 4-methylpiperazin-1-yl, morpholino, 7-azabicyclo[2.2.1]heptan-7-yl, and 3-azabicyclo[3.2.2]nonan-3-yl.
   Suitable "aryl group" may include phenyl, naphthyl, indenyl, anthryl, etc. and this aryl may have one or more suitable substituents such as halogen (e.g. fluoro, chloro, bromo, iodo) ; lower alkoxy (e.g. methoxy, ethoxy, propoxy, tert-butoxy, pentyloxy, hexyloxy, etc.; nitro; amino; protected amino, the species of which may be the same as those mentioned hereinbefore, and so on.

   The preferred "aryl group optionally having one or more suitable substituents" includes phenyl optionally having 1~3 suitable substituents selected from the class consisting of halogen, (C₁-C₄)alkoxy, nitro, amino, (C₁-C₄)alkanoylamino, (C₁-C₄)alkoxy-carbonylamino, (C₁-C₄)alkanesulfonylamino, (C₁-C₄)alkylamino and di(C₁-C₄)alkylamino. The still more preferred, among them, are phenyl, phenyl having chloro, phenyl having methoxy, phenyl having nitro, phenyl having amino, phenyl having acetylamino, phenyl having methoxycarbonylamino, phenyl having methanesulfonylamino, phenyl having methylamino and phenyl having dimethylamino.
   Suitable "heterocyclic group" may include the groups mentioned by way of example for said "nitrogen-containing heterocyclic group";
   unsaturated 3~8-membered (more preferably 5- or 6-membered) monocyclic heterocyclic groups containing 1 oxygen atom, for example furyl;
   unsaturated 3~8-membered (more preferably 5- or 6-membered) monocyclic heterocyclic groups containing 1 oxygen atom and 1 or 2 sulfur atoms, for example dihydrooxathiynyl;
   unsaturated fused heterocyclic groups containing 1 or 2 sulfur atoms, for example benzothienyl and benzodithiynyl; and
   unsaturated fused heterocyclic groups containing 1 oxygen atom and 1 or 2 sulfur atoms, for example benzoxathiynyl.

   The preferred, among these, are unsaturated 3~8-membered monocyclic heterocyclic groups containing 1~4 nitrogen atoms. The still more preferred is pyridyl and the most preferred are 2-pyridyl, 3-pyridyl and 4-pyridyl.
   Suitable "lower alkenyl group having halogen" may include 1-fluorovinyl, 1-bromovinyl, 1-chloro-2-methylvinyl, 1-bromo-1-propenyl, 2-chloro-2-propenyl, 1-iodo-1-butenyl, 1-bromo-2-methyl-1-propenyl, 3-bromo-1,3-butadienyl, 1-chloro-1-pentenyl, 4-chloro-4-pentenyl, 1-bromo-1-hexenyl, among others.
   Suitable "lower alkoxy group" may include methoxy, ethoxy, propoxy, isopropoxy, butoxy, tert-butoxy, pentyloxy and hexyloxy, among others.
   Suitable "halogen" may include fluoro, chloro, bromo and iodo.
   Suitable "leaving group" may include di(lower)alkylamino groups such as dimethylamino, diethylamino, N-ethylpropylamino, dibutylamino, N-pentylhexylamino, etc.; said lower alkoxy groups, said halogen atoms, and lower alkylthio groups such as methylthio, ethylthio, propylthio, butylthio, pentylthio and hexylthio, among others.
   Suitable "unsaturated heterocyclic group" of said "unsaturated heterocyclic group optionally having one or more suitable substituents" may include unsaturated, mono- or polycyclic heterocyclic groups containing at least one hetero atom such as nitrogen, oxygen or sulfur.
   To mention preferred examples, this "unsaturated heterocyclic group" includes unsaturated 3~8-memberd (more preferably 5~7-membered) monocyclic heterocyclic groups containing 1~4 nitrogen atoms, such as azepinyl (e.g. 1H-azepinyl), pyrrolyl, pyrrolinyl, imidazolyl, pyrazolyl, pyridyl, dihydropyridyl (e.g. 1,2-dihydropyridyl, 1,4-dihydropyridyl), tetrahydropyridyl (e.g. 1,2,3,6-tetrahydropyridyl, pyrimidinyl, dihydropyrimidinyl (e.g. 1,2-dihydropyrimidinyl), pyrazinyl, pyridazinyl, dihydropyridazinyl (e.g. 2,3-dihydropyridazinyl, 1,4-dihydropyridazinyl), tetrahydropyridazinyl (e.g. 2,3,4,5-tetrahydropyridazinyl); triazolyl (e.g. 4H-1,2,4-triazolyl, 1H-1,2,3-triazolyl, 2H-1,2,3-triazolyl), tetrazolyl (e.g. 1H-tetrazolyl, 2H-tetrazolyl), etc.;
   unsaturated fused heterocyclic groups containing 1~4 nitrogen atoms, such as indolyl, isoindolyl, indolizinyl, benzimidazolyl, quinolyl, dihydroquinolyl (e.g. 2,3-dihydroquinolyl), isoquinolyl, indazolyl, benzotriazolyl, etc.;
   unsaturated 3-8-membered (more preferably 5 or 6-membered) monocyclic heterocyclic groups containing 1 or 2 oxygen atoms and 1~3 nitrogen atoms, such as oxazolyl, isoxazolyl, dihydroisoxazolyl (e.g. 2,5-dihydroisoxazolyl), oxadiazolyl (e.g. 1,2,4-oxadiazolyl, 1,3,4-oxadiazolyl, 1,2,5-oxadiazolyl), etc.;
   unsaturated fused heterocyclic groups containing 1 or 2 oxygen atoms and 1~3 nitrogen atoms, such as benzoxazolyl, benzoxadiazolyl, etc.;
   unsaturated 3~8-mambered (more preferably 5- or 6-membered) monocyclic heterocyclic groups containing 1 or 2 sulfur atoms and 1~3 nitrogen atoms, such as thiazolyl, dihydrothiazolyl (e.g. 2,3-dihydro-thiazolyl), isothiazolyl, thiadiazolyl (e.g. 1,2,3-thiadiazolyl, 1,2,4-thiadiazolyl, 1,3,4-thiadiazolyl, 1,2,5-thiadiazolyl), dihydrothiazinyl, etc.;
   unsaturated fused heterocyclic groups containing 1 or 2 sulfur atoms and 1~3 nitrogen atoms, such as benzothiazolyl, benzothiadiazolyl (e.g. benzo[d][1,2,3]thiadiazolyl), imidazothiadiazolyl (e.g. 5H-imidazo[2,1-b][1,3,4]thiadiazolyl), etc.;
   unsaturated 3-8-membered (more preferably 5- or 6-membered) monocyclic heterocyclic groups containing 1 or 2 sulfur atoms, such as thienyl, dihydrothiynyl, etc.;
   unsaturated 3-8-membered (more preferably 5- or 6-membered) monocyclic heterocyclic groups containing one oxygen atom, such as furyl etc.;
   unsaturated 3~8-membered (more preferably 5- or 6-membered) monocyclic heterocyclic groups containing one oxygen atom and 1 or 2 sulfur atoms, such as dihydrooxathiynyl etc.;
   unsaturated fused heterocyclic groups containing 1 or 2 sulfur atoms, such as benzothienyl, benzodithiynyl, etc.; and
   unsaturated fused heterocyclic groups containing one oxygen atom and 1 or 2 sulfur atoms, such as benzoxathiynyl and so on.

   The preferred, among these, are unsaturated heterocyclic groups containing at least one nitrogen atom as the hetero atom. The more preferred are unsaturated 3-8-membered monocyclic heterocyclic groups containing 1~4 nitrogen atoms and unsaturated fused heterocyclic groups containing 1 or 2 sulfur atoms and 1~3 nitrogen atoms. The still more preferred are pyridazinyl, dihydropyridazinyl, tetrahydro-pyridazinyl,pyrimidinyl,dihydropyrimidinyl,pyridyl, dihydropyridyl, tetrahydropyridyl, pyrazolyl and imidazothiadiazolyl. The most preferred are pyridazinyl, 2,3-dihydropyridazinyl, 1,4-dihydropyridazinyl, 2,3,4,5-tetrahydropyridazinyl, pyrimidinyl, 1,2-dihydropyrimidinyl, pyridyl, 1,2-dihydropyridyl, 1,4-dihydropyridyl, 1,2,3,6-tetrahydropyridyl, pyrazolyl and imidazo[2,1-b][1,3,4]thiadiazolyl.
   The "unsaturated heterocyclic group" mentioned above may have one or more (preferably 1~4) suitable substituents, for example lower alkyl groups, e.g. methyl, ethyl, propyl, isopropyl, butyl, tert-butyl, pentyl, hexyl, etc., which may optionally have one or more (preferably 1~4) suitable substituents such as those mentioned hereinafter; carboxy(lower)alkenyl groups such as 1-carboxyvinyl, 2-carboxyvinyl, 1-carboxy-2-propenyl, 3-carboxy-2-propenyl, 3-carboxy-2-butenyl, 4-carboxy-2-methyl-2-butenyl, 3-carboxy-1-hexeny, etc.; amino; di(lower)alkylamino such as dimethylamino, N-methylethylamino, dipropylamino, N-butyl-(2-methylbutyl)amino, N-pentylhexylamino; halogen such as fluoro, chloro, bromo and iodo; lower alkoxy such as methoxy, ethoxy, propoxy, isopropoxy, butoxy, tert-butoxy, pentyloxy, hexyloxy, etc.; oxo; hydroxy; cyano; and the acyl group defined below.
   Suitable "acyl group" may include lower alkanoyl groups such as formyl, acetyl, propionyl, butyryl, isobutyryl, pivaloyl, hexanoyl, etc., carboxy and protected carboxy.
   Suitable "protected carboxy" may include esterified carboxy groups, the preferred examples of which are lower alkoxycarbonyl groups such as methoxycarbonyl, ethoxycarbonyl, propoxycarbonyl, tert-butoxycarbonyl, pentyloxycarbonyl, hexyloxycarbonyl, etc.; and
   amidated carboxy groups, the preferred examples of which are carbamoyl and N,N-di (lower) alkylcarbamoyl, the two lower alkyl groups on the nitrogen atom of which may taken together form a 3- through 6-membered ring, such as N,N-dimethylcarbamoyl, N-methyl-N-ethylcarbamoyl, N,N-diethylcarbamoyl, N,N-dipropylcarbamoyl, N-butyl-N-tert-butylcarbamoyl, N,N-dipentylcarbamoyl, N-pentyl-N-hexylcarbamoyl, 1-aziridinylcarbonyl, 1-azetidinylcarbonyl, 1-pryrrolidinylcarbonyl, piperidinocarbonyl, and so on.

   Suitable examples of "suitable substituent" on said "lower alkyl group which may have one or more suitable substituents" may include hydroxy, said halogen, said lower alkoxy, said acyl, etc.
   Suitable examples of the "lower alkyl group having one or more suitable substituents" may include lower alkyl groups having hydroxy and halogen, such as 1-hydroxy-1-chloromethyl, 1-hydroxy-2-chloroethyl, 2-hydroxy-3-fluoropropyl, 2-hydroxy-3,3,3-trichloropropyl, 3-bromo-4-hydroxy-4-iodobutyl, 1-chloro-2-hydroxy-4-fluoropentyl, 3,4-dihydroxy-6-chlorohexyl, etc.;
   hydroxy(lower) alkyl groups such as hydroxymethyl, 2-hydroxyethyl, 2-hydroxypropyl, 3-hydroxypropyl, 1-hydroxy-1-methylethyl, 1-hydroxybutyl, 1-hydroxymethyl-1-methylethyl, 3-hydroxypentyl, 2-hydroxyhexyl, etc.;
   lower alkoxy(lower)alkyl groups such as methoxymethyl, ethoxymethyl, 2-ethoxyethyl, 1-propoxyethyl, 3-isopropoxypropyl, 2-butoxybutyl, 1-tert-butoxymethyl-1-methylethyl, 5-pentyloxypentyl, hexyloxymethyl, 3-hexyloxyhexyl, etc.; and
   acyl (lower) alkyl groups, the examples of which are carboxy(lower)alkyl groups such as carboxymethyl, 2-carboxyethyl, 2-carboxypropyl, 3-carboxypropyl, 2-carboxy-1-methylethyl, 4-carboxybutyl, 1-carboxymethyl-1-methylethyl, 3-carboxypentyl, 2-carboxyhexyl, etc., preferably protected carboxy(lower)alkyl groups such as esterified carboxy(lower)alkyl groups and amidated carboxy(lower)alkyl groups, more preferably lower alkoxycarbonyl(lower)alkyl groups such as methoxycarbonylmethyl, ethoxycarbonylmethyl, 2-methoxycarbonylethyl, 2-ethoxycarbonylethyl, 1-propoxycarbonylethyl, 3-ethoxycarbonylpropyl, 2-butoxycarbonylbutyl, 4-ethoxycarbonylbutyl, 1-tert-butoxycarbonylmethyl-1-methylethyl, 5-pentyloxycarbonylpentyl, hexyloxycarbonylmethyl, 3-hexyloxycarbonylhexyl, etc., carbamoyl(lower)alkyl groups such as carbamoylmethyl, 2-carbamoylethyl, 3-carbamoylpropyl, 2-carbamoyl-1-methylethyl, 4-carbamoylbutyl, 1-carbamoylmethyl-1-methylethyl, 5-carbamoylpentyl, 3-carbamoylhexyl, etc., and N,N-di(lower)alkylcarbamoyl(lower)alkyl groups, the two lower alkyl groups on the nitrogen atom of which may taken together form a 3-6-membered ring, such as N,N-dimethylcarbamoylmethyl, 2-(N,N-dimethyl-carbamoyl)ethyl, 2-(N-methyl-N-ethylcarbamoyl)ethyl, 3-(N-methyl-N-ethylcarbamoyl)propyl, 2-(N,N-dipropylcarbamoyl)-1-methylethyl, 4-(N,N-dipropyl-carbamoyl)butyl, 1-(N,N-dimethylcarbamoyl)methyl-1-methylethyl, 5-(N-pentyl-N-hexylcarbamoyl)pentyl, 3- (N-pentyl-N-hexylcarbamoyl)hexyl, (1-aziridinylcarbonyl)methyl, 2-(1-azetidinylcarbonyl)ethyl, 2-(piperidinocarbonyl)ethyl, 3-(1-pyrrolidinylcarbonyl)propyl, 2-(piperidinocarbonyl)-1-methylethyl, 4-(1-azetidinylcarbonyl)-butyl, 1-(1-aziridinylcarbonyl)methyl-1-methylethyl, 3-(1-pyrrolidinylcarbonyl)pentyl, 6-(piperidinocarbonyl)hexyl, and so on.

   The preferred substituent on said "unsaturated heterocyclic group" may include lower alkyl, lower alkyl *having* hydroxy and halogen, hydroxy (lower) alkyl, lower alkoxy(lower)alkyl, carboxy(lower)alkyl, lower alkoxycarbonyl (lower) alkyl, carbamoyl(lower)alkyl, N,N-di(lower)alkylcarbamoyl(lower)alkyl, the two lower alkyl groups on the nitrogen atom of which may taken together form a 3-6-membered ring, carboxy(lower)-alkenyl, di(lower)alkylamino, halogen, lower alkoxy, oxo, carboxy, lower alkoxycarbonyl, lower alkanoyl, amino, cyano and hydroxy. Among these, the more preferred are(C₁-C₄)alkyl, (C₁-C₄)alkyl having hydroxy and halogen, hydroxy(C₁-C₄)alkyl, (C₁-C₄)alkoxy(C₁-C₄)alkyl, carboxy(C₁-C₄)alkyl, (C₁-C₄)alkoxycarbonyl(C₁-C₄)alkyl, carbamoyl(C₁-C₄)alkyl, N,N-di(C₁-C₄)alkylcarbamoyl-(C₁-C₄)alkyl, piperidinocarbonyl(C₁-C₄)alkyl, carboxy(C₂-C₄)alkenyl, di(C₁-C₄)alkylamino, halogen, (C₁-C₄)alkoxy, oxo, carboxy, (C₁-C₄)alkoxycarbonyl, (C₁-C₄)alkanoyl, amino, cyano and hydroxy. The most preferred are methyl, propyl, 2-hydroxy-3,3,3-trichloropropyl, 2-hydroxyethyl, 3-hydroxypropyl, 2-ethoxyethyl, 2-carboxyethyl, 3-carboxypropyl, 4-carboxybutyl, methoxycarbonylmethyl, 2-methoxycarbonylethyl, 3-ethoxycarbonylpropyl, 4-ethoxycarbonylbutyl, 2-carbamoylethyl, 2-(N,N-dimethylcarbamoyl)ethyl, 2-(piperidino-carbonyl)ethyl,2-carboxyvinyl, dimethylamino, chloro, methoxy, oxo, carboxy, ethoxycarbonyl, methoxycarbonyl, acetyl, amino, cyano and hydroxy.
   The "unsaturated heterocyclic group" of said "unsaturated heterocyclic group optionally having one or more suitable substituents" may have any of the following substituents, not to speak of the substituent groups mentioned hereinbefore, in the number of one or more (preferably 1~4).
   Such substituents may include amino(lower)alkyl; lower alkylamino(lower)alkyl;
   carboxy(lower)alkylamino(lower)alkyl; protected carboxy(lower)alkylamino(lower)alkyl; lower alkylamino(lower)alkyl having hydroxy and aryloxy;
   protected amino(lower)alkyl; cyano(lower)alkyl; cyano(higher)alkyl; lower alkyl having a heterocyclic group which may have one or more suitable substituents;
   higher alkyl having a heterocyclic group which may have one or more suitable substituents; ar(lower)alkyl; lower alkenyl; heterocyclic groups optionally having one or more suitable substituents; cyclo(lower)alkyl optionally having one or more suitable substituents;
   or cyclo(lower)alkenyl optionally having one or more suitable substituents.

   The substituents mentioned above are now explained.
   Suitable "amino(lower)alkyl" may include aminomethyl, 1-aminoethyl, 2-aminoethyl, 2-aminopropyl, 3-aminobutyl, 2-amino-1,1-dimethylethyl, 5-aminopentyl, 1-aminohexyl, etc. and, among these, amino(C₁-C₄)alkyl is preferred and 2-aminoethyl is more preferred.
   Suitable "lower alkylamino(lower)alkyl" may include mono- or di(lower)alkylamino(lower)alkyl groups such as methylaminomethyl, 2-(ethylamino)ethyl, 3-(propylamino)propyl, 2-(propylamino)butyl, 2-(t-butylamino)-1,1-dimethylethyl, 4-pentylaminopentyl, 6-hexylaminohexyl, dimethylaminomethyl, 2-dimethylaminoethyl, 1-(N-methylethylamino)ethyl, 1-dimethylaminopropyl, 2-diethylaminopropyl, 3-dimethylaminopropyl, 3-(N-propylbutylamino)butyl, 4-dimethylaminobutyl, 2-dibutylamino-1,1-dimethylethyl, 4-dipentylaminopentyl, 6-(N-pentylhexylamino)hexyl, and so on. Among these, di(lower)alkylamino(lower)alkyl groups are preferred and di (C₁-C₄)alkylamino (C₁-C₄)alkyl groups are more preferred. The most preferred are 2-dimethylaminoethyl, 3-dimethylaminopropyl and 4-dimethylaminobutyl.
   Suitable "carboxy(lower)alkylamino(lower)alkyl" may include carboxymethylaminomethyl, 2-(carboxymethylamino)ethyl, 2-(1-carboxyethyl-amino)ethyl, 3-(2-carboxypropylamino)propyl, 2- (3-carboxypropylamino)butyl, 2-(2-carboxy-1,1-dimethylethylamino)-1,1-dimethylethyl, 4-(5-carboxypentylamino)pentyl, 6- (3-carboxyhexyl-amino)hexyl, etc. Among these, carboxy(C₁-C₄)-alkylamino(C₁-C₄)alkyl groups are preferred, with 2-(carboxymethylamino)ethyl being the most preferred species.
   Suitable "protected carboxy" of the "protected carboxy(lower)alkylamino(lower)alkyl" may include esterified carboxy and the ester moiety of this esterified carboxy includes lower alkyl esters optionally having suitable substituents (e.g. methyl ester, ethyl ester, propyl ester, isopropyl ester, butyl ester, isobutyl ester, t-butyl ester, pentyl ester, hexyl ester, 1-cyclopropylethyl ester, etc.), lower alkanoyloxy(lower)alkyl esters (e.g. acetoxymethyl ester, propionyloxymethyl ester, butyryloxymethyl ester, valeryloxymethyl ester, pivaloyloxymethyl ester, 1-acetoxyethyl ester, 1-propionyloxyethyl ester, pivaloyloxymethyl ester, 2-propionyloxyethyl ester, hexanoyloxymethyl ester, etc.), lower alkanesulfonyl(lower)alkyl esters (e.g. 2-mesylethyl ester etc.) or mono(or di-or tri-)halo(lower)alkyl esters (e.g. 2-iodoethyl ester, 2,2,2-trichloroethyl ester, etc.); lower alkenyl esters (e.g. vinyl ester, allyl ester, etc.); lower alkynyl esters (e.g. ethynyl ester, propynyl ester, etc.); ar(lower)alkyl esters optionally having suitable substituents [e.g. benzyl ester, 4-methoxybenzyl ester, 4-nitrobenzyl ester, phenethyl ester, trityl ester, benzhydryl ester, bis(methoxyphenyl)methyl ester, 3,4-dimethoxybenzyl ester, 4-hydroxy-3,5-di-t-butylbenzyl ester, etc.]; and aryl esters optionally having suitable substituents (e.g. phenyl ester, 4-chlorophenyl ester, tolyl ester, 4-t-butylphenyl ester, xylyl ester, mesityl ester, cumenyl ester, etc.).
   Suitable "protected carboxy(lower)-alkylamino(lower)alkyl" may include esterified carboxy(lower)alkylamino(lower)alkyl groups, and the preferred, among them, are lower alkoxycarbonyl(lower)alkylamino(lower)alkyl groups, such as methoxycarbonylmethylaminomethyl, 2-(ethoxy-carbonylmethylamino)ethyl, 2-(1-ethoxycarbonyl-ethylamino)ethyl, 3-(2-propoxycarbonylpropyl-amino)propyl, 2-(3-butoxycarbonylpropylamino)butyl, 2-(2-t-butoxycarbonyl-1,1-dimethylethylamino)-1,1-d imethylethyl, 4-(5-pentyloxycarbonylpentyl-amino)pentyl, 6-(3-hexyloxycarbonylhexylamino)hexyl, and so on The more preferred are (C₁-C₄)alkoxy-carbonyl(C₁-C₄)alkylamino(C₁-C₄)alkyl, with 2-(ethoxycarbonylmethylamino)ethyl being the most preferred.
   Suitable "lower alkylamino(lower)alkyl having hydroxy and aryloxy" may include said "lower alkylamino(lower)alkyl" which has "hydroxy" and "aryloxy" (e.g. phenoxy, tolyloxy, naphthyloxy, etc.), and the suitable examples of which are 1-(1-naphthyloxy)-1-hydroxymethylaminomethyl, 2-(1-hydroxy-2-phenoxyethylamino)ethyl, 2-[2-hydroxy-3-(1-naphthyloxy)propylamino]ethyl, 2-[4-hydroxy-3-(p-tolyloxy)butylamino]propyl, 2-[4-hydroxy-1-(2-naphthyloxy)butylamino]-1,1-dimet hylethyl, 4-[1-hydroxy-5-(1-naphthyloxy)-pentylamino]pentyl and 6-[2-hydroxy-4-(2-naphthyloxy)hexylamino]hexyl. Among these, the preferred are (C₁-C₄)alkylamino(C₁-C₄)alkyl groups having hydroxy and naphthyloxy, with 2-[2-hydroxy-3-(1-naphthyloxy)propylamino]ethyl being the most preferred.
   Suitable "protected amino(lower)alkyl" may include acylamino(lower)alkyl groups.
   The suitable example of said acylamino may be lower alkanoylamino (e.g. formylamino, acetylamino, propionylamino, hexanoylamino, pivaloylamino, etc.), mono(or di- or tri-)halo(lower)alkanoylamino (e.g. chloroacetylamino, trifluoroacetylamino, etc.), lower alkoxycarbonylamino (e.g. methoxycarbonylamino, ethoxycarbonylamino, t-butoxycarbonylamino, t-pentyloxycarbonylamino, hexyloxycarbonylamino, etc.), mono(or di- or tri-)halo(lower)alkoxy-carbonylamino (e.g. chloromethoxycarbonylamino, dichloroethoxycarbonylamino, trichloroethoxy-carbonylamino, etc.), aroylamino (e.g. benzoylamino, toluoylamino, xyloylamino, naphthoylamino, etc.), ar(lower)alkanoylamino such as phenyl(lower)-alkanoylamino (e.g. phenylacetylamino, phenyl-propionylamino, etc.), aryloxycarbonylamino (e.g. phenoxycarbonylamino, naphthyloxycarbonylamino, etc.), aryloxy(lower)alkanoylamino such as phenoxy(lower)-alkanoylamino (e.g. phenoxyacetylamino, phenoxypropionylamino, etc.), arylglyoxyloylamino (e.g. phenylglyoxyloylamino, naphthylglyoxyloylamino, etc.), ar(lower)alkoxycarbonylamino optionally having suitable substituents, such as phenyl(lower)alkoxycarbonylamino optionally having nitro or lower alkoxy (e.g. benzyloxycarbonylamino, phenethyloxycarbonyl-amino, p-nitrobenzyloxycarbonylamino, p-methoxybenzyloxycarbonylamino, etc.), thienylacetylamino, imidazolylacetylamino, furylacetylamino, tetrazolylacetylamino, thiazolylacetylamino, thiadiazolylacetylamino, thienylpropionylamino, thiadiazolylpropionylamino, lower alkylsulfonylamino (e.g. methylsulfonylamino, ethylsulfonylamino, propylsulfonylamino, isopropylsulfonylamino, pentylsulfonylamino, butylsulfonylamino, etc.), arylsulfonylamino (e.g. phenylsulfonylamino, tolylsulfonylamino, xylylsulfonylamino, naphthylsulfonylamino, etc.), ar(lower)alkylsulfonylamino such as phenyl(lower)-alkylsulfonylamino (e.g. benzylsulfonylamino, phenethylsulfonylamino, benzhydrylsulfonylamino, etc.), and imides (e.g. 1,2-cyclohexanedicarboximido, succinimido, phthalimido, etc.), among others.
   The preferred examples of said "protected amino(lower)alkyl" may include imido(lower)alkyl such as phthalimidomethyl, 2-phthalimidoethyl, 1-(1,2-cyclohexanedicarboximido)ethyl, 2-succinimidopropyl, 3-phthalimidobutyl, 2-(1,2-cyclohexanedi-carboximido)-1,1-dimethylethyl, 5-phthalimidopentyl, 1-phthalimidohexyl, and so on. The more preferred are imido(C₁-C₄)alkyl, with 2-phthalimidoethyl being the most preferred.
   Suitable "cyano(lower)alkyl" may include cyanomethyl, 1-cyanoethyl, 2-cyanoethyl, 3-cyanopropyl, 2-cyanobutyl, 4-cyanobutyl, 2-cyano-1,1-dimethylethyl, 4-cyanopentyl, 5-cyanopentyl, 6-cyanohexyl, etc.; the preferred are cyano(C₁-C₆)alkyl groups and the most preferred are cyanomethyl, 2-cyanoethyl, 3-cyanopropyl, 4-cyanobutyl, 5-cyanopentyl and 6-cyanohexyl.
   Suitable "cyano(higher)alkyl" may include 7-cyanoheptyl, 8-cyanooctyl, 4-cyanooctyl, 8-cyano-3-methylheptyl, 9-cyanononyl, 1-cyanononyl, 10-cyanodecyl, 8-cyanoundecyl, 12-cyanododecyl, 11-cyano-4-methylundecyl, 13-cyanotridecyl, 6-cyanotetradecyl, 15-cyanopentadecyl, 12-cyanohexadecyl, 17-cyanoheptadecyl, 4-cyanooctadecyl, 19-cyanononadecyl, 1-cyano-12-ethylheptadecyl, 20-cyanoeicosyl, etc.; among these, cyano (C₇-C₁₆) alkyl groups are preferred and 7-cyanoheptyl, 8-cyanooctyl, 9-cyanononyl, 10-cyanodecyl and 12-cyanododecyl are more preferred.
   Suitable "lower alkyl" may include straight-chain or branched-chain alkyl groups, for example methyl, ethyl, propyl, isopropyl, butyl, t-butyl, pentyl and hexyl, among others.
   Suitable "lower alkenyl" may include straight-chain or branched-chain alkenyl groups, for example vinyl, allyl, 2-butenyl, 2-methyl-2-propenyl, 4-pentenyl, 3-hexenyl, etc; among these, (C₂-C₄) alkenyl groups are preferred, with vinyl being more preferred.
   Suitable "lower alkyl" of said "lower alkyl having a heterocyclic group which may have one or more suitable substituents" may include the groups mentioned hereinbefore, although (C₁-C₆)alkyl are preferred and methyl, ethyl, propyl, butyl, pentyl and hexyl are the most preferred.
   Suitable "higher alkyl" of said "higher alkyl having a heterocyclic group which may have one or more suitable substituents" may include heptyl, octyl, 3-methylheptyl, nonyl, 2,6-dimethylheptyl, decyl, undecyl, dodecyl, 4-methyldodecyl, tridecyl, tetradecyl, pentadecyl, hexadecyl, heptadecyl, octadecyl, nonadecyl, 12-ethylheptadecyl, eicosyl, and so on. Among these, (C₇-C₁₆)alkyl groups are preferred and heptyl, octyl, nonyl, decyl and dodecyl are more preferred.
   Suitable "heterocyclic group" of said "lower alkyl group having a heterocyclic group which may have one or more suitable substituents" and of said "higher alkyl group having a heterocyclic group which may have one or more suitable substituents" may include saturated or unsaturated, monocyclic or polycyclic heterocyclic groups containing at least one hetero atom typically selected from among O, S and N. The particularly preferred heterocyclic group includes unsaturated 3-8-membered monocyclic heterocyclic groups containing 1~4 nitrogen atoms, such as pyrrolyl, pyrrolinyl, imidazolyl, pyrazolyl, pyridyl and its N-oxide, pyrimidyl, pyrazinyl, pyridazinyl, triazolyl (e.g. 4H-1,2,4-triazolyl, 1H-1,2,3-triazolyl, 2H-1,2,3-triazolyl, etc.), tetrazolyl (e.g. 1H-tetrazolyl, 2H-tetrazolyl, etc.), dihydrotriazinyl (e.g. 4,5-dihydro-1,2,4-triazinyl, 2,5-dihydro-1,2,4-triazinyl, etc.), etc.;
   saturated 3-8-membered heteromonocyclic groups containing 1~4 nitrogen atoms, such as pyrrolidinyl, imidazolidinyl, piperidyl (e.g. piperidino etc.), piperazinyl, etc.;
   unsaturated fused heterocyclic groups containing 1-5 nitrogen atoms, such as indolyl, isoindolyl, indolizinyl, benzimidazolyl, quinolyl, isoquinolyl, indazolyl, benzotriazolyl, tetrazolopyridyl, tetrazolopyridazinyl (e.g. tetrazolo[1,5-b]-pyridazinyl etc.), dihydrotriazolopyridazinyl, etc.;
   unsaturated 3-8-membered heteromonocyclic groups containing 1 or 2 oxygen atoms and 1~3 nitrogen atoms, such as oxazolyl, isoxazolyl, oxadiazolyl (e.g. 1,2,4-oxadiazolyl, 1,3,4-oxadiazolyl, 1,2,5-oxadiazolyl, etc.), etc.;
   saturated 3~8-membered heteromonocyclic groups containing 1 or 2 oxygen atoms and 1~3 nitrogen atoms, such as morpholinyl, oxazolidinyl (e.g. 1,3-oxazolidinyl etc.), etc.;
   unsaturated fused heterocyclic groups containing 1 or 2 oxygen atoms and 1~3 nitrogen atoms, such as benzoxazolyl, benzoxadiazolyl, etc.;
   unsaturated 3~8-membered heteromonocyclic groups containing 1 or 2 sulfur atoms and 1~3 nitrogen atoms, such as 1,3-thiazolyl, 1,2-thiazolyl, thiazolinyl, thiadiazolyl (e.g. 1,2,4-thiadiazolyl, 1,3,4-thiadiazolyl, 1,2,5-thiadiazolyl, 1,2,3-thiadiazolyl, etc.), etc.;
   saturated 3~8-membered heteromonocyclic groups containing 1 or 2 sulfur atoms and 1~3 nitrogen atoms, such as thiazolidinyl etc.,
   unsaturated 3~8-membered heteromonocyclic groups containing one sulfur atom, such as thienyl etc.;
   unsaturated fused heterocyclic groups containing 1 or 2 sulfur atoms and 1~3 nitrogen atoms, such as benzothiazolyl, benzothiadiazolyl, etc.;
   unsaturated 3~8-membered heteromonocyclic groups containing 1 or 2 oxygen atoms, such as furyl, pyranyl, dioxolyl, etc.;
   saturated 3~8-membered heteromonocyclic groups containing 1 or 2 oxygen atoms, such as oxolanyl, tetrahydropyranyl (e.g. tetrahydro-2H-pyran-2-yl etc.), dioxolanyl, etc.;
   unsaturated fused heterocyclic groups containing 1 or 2 oxygen atoms, such as isobenzofuranyl, chromenyl (e.g. 2H-chromen-3-yl etc.), dihydrochromenyl (e.g. 3,4-dihydro-2H-chromen-4-yl etc.), and so on.

   The preferred examples of said "heterocyclic group" include unsaturated 3~8-membered heteromonocyclic groups containing 1~4 nitrogen atoms; saturated 3~8-membered heteromonocyclic groups containing 1~4 nitrogen atoms; saturated 3~8-membered heteromonocyclic groups containing 1 or 2 oxygen atoms and 1~3 nitrogen atoms; and saturated 3~8-membered heteromonocyclic groups containing 1 or 2 oxygen atoms. Among these, the preferred are pyridyl, tetrazolyl, piperidyl, piperazinyl, morpholinyl, oxazolidinyl and tetrahydropyranyl, and the more preferred are 4-pyridyl, 1H-tetrazol-5-yl, piperidino, 1-piperazinyl, morpholino, 1,3-oxazolidin-5-yl and tetrahydro-2H-pyran-2-yl.
   The "heterocyclic group" mentioned above may have 1 or more (preferably 1~3) suitable substituents [such as hydroxy(lower)alkyl (e.g. hydroxymethyl, 2-hydroxyethyl, 1-hydroxypropyl, 4-hydroxybutyl, 2-hydroxy-1,1-dimethylethyl, 3-hydroxypentyl, 6-hydroxyhexyl, etc.), aryl which may have lower alkoxy (e.g. phenyl, naphthyl, 2-methoxyphenyl, 2-methoxynaphthyl, 3-ethoxyphenyl, 4-propoxyphenyl, 2-butoxyphenyl, 5-propoxynaphthyl, 3-t-butoxyphenyl, 4-pentyloxyphenyl, 2-hexyloxyphenyl, etc.), oxo, etc.]. The preferred among such "suitable substituents" are hydroxy(C₁-C₄)alkyl, phenyl having (C₁-C₄)alkoxy, and oxo, and the more preferred are 2-hydroxyethyl, 2-methoxyphenyl and oxo.
   Suitable "heterocyclic group" of said "heterocyclic group optionally having one or more suitable substituents" may include the "heterocyclic groups" mentioned for said "lower alkyl having a heterocyclic group which may have one or more suitable substituents" and "higher alkyl having a heterocyclic group which may have one or more suitable substituents". Among them, the preferred are unsaturated fused heterocyclic groups containing one or more oxygen atoms and the more preferred is dihydrochromenyl, with 3,4-dihydro-2H-chromen-4-yl being the most preferred species.
   This "heterocyclic group" may have one or more (preferably 1~4) suitable substituents [such as said lower alkyl, hydroxy, cyano, etc., preferably (C₁-C₄)alkyl, hydroxy and cyano, most preferably methyl, hydroxy and cyano].
   Suitable "ar(lower)alkyl" may include mono-, di-or triphenyl(lower)alkyl (e.g. benzyl, phenethyl, 2-phenylpropyl, 4-phenylbutyl, 2-phenyl-1,1-dimethylethyl, 1-phenylpentyl, 6-phenylhexyl, benzhydryl, trityl, etc.); the preferred, among these, are phenyl(C₁-C₄)alkyl groups, with benzyl being the most preferred.
   Suitable "nitrogen-containing heterocyclic group" of said "nitrogen-containing heterocyclic group optionally having one or more suitable substituents" may include those heterocyclic groups, among the various "heterocyclic groups" mentioned above, which contain at least one nitrogen atom as a ring atom, and this "nitrogen-containing heterocyclic group" may have one or more (preferably 1~3) suitable substituents [such as saidhydroxy (lower) alkyl, said aryl optionally having lower alkoxy, oxo, etc.].
   Suitable "tetrazolyl(lower)alkyl" may include 1H-tetrazol-5-ylmethyl, 2-(1H-tetrazol-5-yl)ethyl, 3-(1H-tetrazol-5-yl)propyl, 4-(1H-tetrazol-5-yl)-butyl, 2-(2H-tetrazol-2-yl)-1,1-dimethylethyl, 4-(1H-tetrazol-l-yl)pentyl, 5-(1H-tetrazol-5-yl)-pentyl, 6-(1H-tetrazol-5-yl)hexyl, etc. and, among these, tetrazolyl(C₁-C₆)alkyl groups are preferred. The more preferred are (1H-tetrazol-5-yl)methyl, 2-(1H-tetrazol-5-yl)ethyl, 3-(lH-tetrazol-5-yl)-propyl, 4-(1H-tetrazol-5-yl)butyl, 5-(1H-tetrazol-5-yl)pentyl and 6-(lH-tetrazol-5-yl)hexyl.
   Suitable "tetrazolyl(higher)alkyl" may include 7-(lH-tetrazol-5-yl)heptyl, 8-(1H-tetrazol-5-yl)-octyl, 4-(1H-tetrazol-1-yl)octyl, 8-(1H-tetrazol-5-yl)-3-methylheptyl, 9-(lH-tetrazol-5-yl)nonyl, 1-(1H-tetrazol-1-yl)nonyl, 10-(1H-tetrazol-5-yl)-decyl, 8-(1H-tetrazol-5-yl)undecyl, 12-(1H-tetrazol-5-yl)dodecyl, 11-(1H-tetrazol-5-yl)-4-methylundecyl, 13-(1H-tetrazol-5-yl)tridecyl, 6-(lH-tetrazol-5-yl)-tetradecyl, 15-(lH-tetrazol-5-yl)pentadecyl, 12-(1H-tetrazol-5-yl)hexadecyl, 17-(1H-tetrazol-1-yl)-heptadecyl, 4-(lH-tetrazol-5-yl)octadecyl, 19-(1H-tetrazol-5-yl)nonadecyl, 1-(1H-tetrazol-1-yl)-12-ethylheptadecyl, 20-(1H-tetrazol-5-yl)eicosyl, etc.; among these, tetrazolyl(C₇-C₁₆)alkyl groups are preferred. The more preferred are 7-(1H-tetrazol-5-yl)heptyl, 8-(1H-tetrazol-5-yl)-octyl, 9-(1H-tetrazol-5-yl)nonyl, 10-(1H-tetrazol-5-yl)decyl and 12-(1H-tetrazol-5-yl)dodecyl.
   Suitable "cyclo(lower)alkyl" may include cyclo (C₃-C₆) alkyl groups such as cyclopropyl, cyclobutyl, cyclopentyl, cyclohexyl, cycloheptyl, cyclooctyl, etc.; the preferred, among these, are cyclo(C₅-C₇)alkyl groups such as cyclopentyl, cyclohexyl, cycloheptyl, etc.
   This "cyclo(lower)alkyl" may have one or more (preferably 1~3) suitable substituents selected from the class consisting of acyl(lower)alkyl and acyl(lower)alkylidene, among others.
   Suitable "cyclo(lower)alkenyl" may include cyclo(C₃-C₈)alkenyl groups such as cyclopropenyl, cyclobutenyl, cyclopentenyl, cyclohexenyl, cycloheptenyl, cyclooctenyl, etc., and the preferred among these are cyclo(C₅-C₇)alkenyl groups such as cyclopentenyl, cyclohexenyl and cycloheptenyl. The more preferred is cyclohexenyl or cycloheptenyl.
   This "cyclo(lower)alkenyl" may have one or more (preferably 1~3) suitable substituents such as those mentioned for said "cyalo(lower)alkyl".
   Suitable examples of the above "acyl(lower)alkyl" may include carboxy(lower)alkyl groups such as carboxymethyl, 2-carboxyethyl, 3-carboxypropyl, 1-carboxymethylethyl, 4-carboxybutyl, 2-carboxymethyl-2-methylethyl, 5-carboxypentyl, 3-carboxyhexyl, etc. and lower alkanoyl(lower)alkyl groups such as acetylmethyl, formylmethyl, 2-acetylethyl, 2-propionylpropyl, 4-butyrylbutyl, 3-pentanoylpentyl, 6-hexanoylhexyl, etc. The preferred, among these, are carboxy(C₁-C₄)alkyl or (C₁-C₄)alkanoyl (C₁-C₄)alkyl, with carboxymethyl, 2-carboxyethyl, 3-carboxypropyl or acetylmethyl being more preferred.
   As other suitable examples of said "acyl(lower)alkyl", there can be mentioned protected carboxy(lower)alkyl groups, and the preferred, among these, are esterified carboxy(lower)alkyl groups. The more preferred are lower alkoxycarbonyl(lower)alkyl groups such as methoxycarbonylmethyl, ethoxycarbonylmethyl, 2-ethoxycarbonylethyl, 1-propoxycarbonylpropyl, 2-isopropoxycarbonylpropyl, butoxycarbonylmethyl, t-butoxycarbonylmethyl, 4-isobutoxycarbonylbutyl, 3-pentyloxycarbonylpentyl, 6-hexyloxycarbonylhexyl, (1-cyclopropylethoxy-carbonyl)methyl, etc. and phenyl(lower)alkoxycarbonyl(lower)alkyl groups such as benzyloxy-carbonylmethyl, 2-benzyloxycarbonylethyl, 1-phenethyloxycarbonylethyl, 3-benzyloxycarbonylpropyl, 2-benzyloxycarbonylbutyl, 2-phenethyloxycarbonylmethyl-2-methylethyl, 3-benzyloxycarbonylpentyl, 6-benzyloxycarbonylhexyl, etc. The more preferred are (C₁-C₄)alkoxycarbonyl (C₁-C₄) alkyl or phenyl(C₁-C₄) alkoxycarbonyl(C₁-C₄) alkyl, and the particularly preferred species are methoxycarbonylmethyl, ethoxycarbonylmethyl, t-butoxycarbonylmethyl, 2-benzyloxycarbonylethyl and 3-benzyloxycarbonylpropyl.
   Suitable examples of said "acyl (lower) alkylidene" may include carboxy(lower)alkylidene groups such as carboxymethylene, 2-carboxyethylidene, 2-carboxypropylidene, 4-carboxybutylidene, 5-carboxypentylidene, 3-carboxyhexylidene, etc., and the preferred, among these, are carboxy (C₁-C₄) alkylidene groups, with carboxymethylene being more preferred.
   As other suitable examples of the "acyl(lower)alkylidene", there can be mentioned protected carboxy(lower)alkylidene groups, preferably esterified carboxy(lower)alkylidene groups, and more preferably lower alkoxycarbonyl(lower)alkylidene groups such as methoxycarbonylmethylene, ethoxycarbonylmethylene, 2-ethoxycarbonylethylidene, 1-propoxycarbonylpropylidene, 2-isopropoxycarbonyl-propylidene, butoxycarbonylmethylene, t-butoxy-carbonylmethylene, 4-isobutoxycarbonylbutylidene, 3-pentyloxycarbonylpentylidene, 6-hexyloxycarbonylhexylidene, (1-cyclopropylethoxycarbonyl) methylene, etc. The still more preferred are (C₁-C₄) alkoxy-carbonyl(C₁-C₄)alkylidene groups and the particularly preferred species are methoxycarbonylmethylene, ethoxycarbonylmethylene and t-butoxycarbonyl-methylene.
   Among the various species of pyrazolopyridine compound (I) described above, the following compound is particularly preferred for the practice of this invention.
   3-[2-(Thiazol-2-ylmethyl)-3-oxo-2,3-dihydro-pyridazin-6-yl]-2-phenylpyrazolo[1,5-a]pyridine
2. A compound selected from among pyrazolopyrazine compounds of the following general formula (II) or salts thereof: 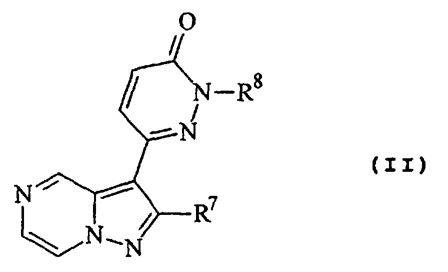 [wherein R⁷ is aryl optionally having one or more suitable substituents; R⁸ is hydrogen, lower alkyl, cyclo(lower)alkyl, lower alkyl substituted by cyclo(lower)alkyl, ar(lower)alkyl, heterocyclic group, or lower alkyl substituted by heterocyclic group].
   Hereat, the selection of a compound is carried out by judiciously selection of a suitable compound having preferred profile after estimating strength of adenosine A₁ and A₂ₐ-receptor antagonizing activity by the above method of "Estimation of Adenosine A₁ and A₂ₐ-receptor antagonizing activity"
   Suitable salts of such pyrazolopyrazine compounds include the same kinds of salts as mentioned by way of example for said pyrazolopyridine compound (I).
   The following comments on the various definitions of the compound (II) are relevant to the preferred examples thereof.
   Suitable "aryl" and "aryl optionally having one or more suitable substituents" may include the same groups as mentioned by way of example for the pyrazolopyridine compound (I). Among them, the preferred examples are phenyl, phenyl having chloro, phenyl having fluoro, and phenyl having methoxy.
   Suitable "lower alkyl group" may include the same groups as mentioned by way of example for the compound (I), and among them, methyl, ethyl, propyl and isopropyl are preferred.
   Suitable "cyclo (lower) alkyl group" may include the same groups as mentioned by way of example for the compound (I); among them, cyclo(C₅-C₇)alkyl groups such as cyclopentyl, cyclohexyl and cycloheptyl are preferred.
   Suitable "heterocyclic group may " include the same groups (inclusive of nitrogen-containing heterocyclic groups) as mentioned by way of example for the compound (I), and the preferred, among them, are saturated 5-or 6-membered heteromonocyclic groups containing one oxygen atom, such as trihydrofuryl, tetrahydropyranyl, etc., and unsaturated 5- or 6-membered heteromonocyclic groups containing one nitrogen, oxygen or sulfur atom, such as pyridyl, furyl, thienyl, and so on.
   Suitable "ar(lower)alkyl" may include the same groups as mentioned by way of example for the compound (I).
   The "ar(lower)alkyl group" mentioned just above may have one or more (preferably 1~3) suitable substituents typically selected from among said lower alkyl, said halogen, hydroxy, and said lower alkoxy.
   Suitable "cyclo(lower)alkyl" of said "lower alkyl substituted by cyclo(lower)alkyl" may include the same groups as mentioned hereinbefore as species of "cyclo(lower)alkyl".
   Suitable "heterocyclic group" of said "lower alkyl substituted by heterocyclic group" may include the same groups as mentioned hereinbefore in the description of "heterocyclic group".
   Suitable "lower alkyl" of said "lower alkyl substituted by cyclo(lower)alkyl" and of said "lower alkyl substituted by heterocyclic group" may include the same groups as mentioned hereinbefore in the description of compound (I), and the preferred, among them, are methyl, ethyl, propyl, butyl, pentyl and hexyl.
3. A compound selected from among xanthine compounds of the following general formula (III) or salts thereof: 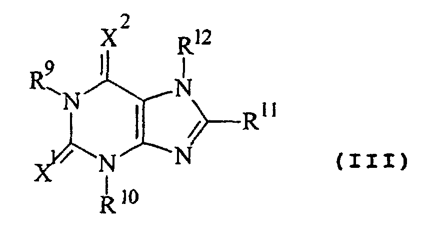 [wherein R⁹, R¹⁰ and R¹² each is hydrogen, a lower aliphatic hydrocarbon group optionally having one or more suitable substituents, higher alkyl optionally having one or more suitable substituents or ar(lower)alkyl optionally having one or more suitable substituents; R¹¹ is hydrogen; alicyclic, aryl, heterocyclic, alicyclic(lower)alkyl, ar(lower)alkyl or heterocyclic(lower)alkyl group, each of which may have one or more suitable substituents; or a group of the formula: 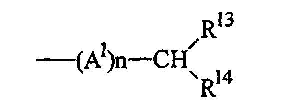 (wherein R¹³ and R¹⁴ each is alicyclic group optionally having one or more suitable substituents or aryl optionally having one or more suitable substituents; A¹ is lower alkylene; and n is 0 or 1) ; and X¹ and X² each is an oxygen atom or a sulfur atom].
   Hereat, the selection of a compound is carried out by judiciously selection of a suitable compound having preferred profile after estimating strength of adenosine A₁ and A₂ₐ-receptor antagonizing activity by the above method of "Estimation of Adenosine A₁ and A₂ₐ-receptor antagonizing activity"
   Suitable salts of such xanthine compounds may include the same kinds of salts as mentioned hereinbefore for the pyrazolopyridine compound (I).
   The xanthine compound (III) includes all the compounds described in EP 0386675, EP 0415456, JP H2-247180, and WO 90/12797. By the same token, the groups mentioned for compound (III) herein apply to all the corresponding groups of said various compounds.
   The following comments on the various definitions of compound (III) are directed to the particularly preferred examples thereof.
   Suitable "lower aliphatic hydrocarbon group" of said "lower aliphatic hydrocarbon group optionally having one or more suitable substituents" may include the same lower alkyl, lower alkenyl and lower alkynyl as mentioned for the pyrazolopyridine compound (I).
   The "lower aliphatic hydrocarbon group" mentioned above may have one or more (preferably 1~3) suitable substituents typically selected from among hydroxy, amino, the halogen mentioned for compound (I) and the aryl mentioned for compound (I).
   As preferred examples of said "lower aliphatic hydrocarbon group", there can be mentioned (C₁-C₄)alkyl, (C₂-C₄)alkenyl and (C₂-C₄)alkynyl; the more preferred, among these, are (C₁-C₄)alkyl groups, with propyl being the most preferred.
   Suitable "higher alkyl" of said "higher alkyl optionally having one or more suitable substituents" may include the same groups as mentioned for compound (I), and this "higher alkyl" may have one or more (preferably 1~3) suitable substituents such as those mentioned for said "lower aliphatic hydrocarbon group".
   Suitable "ar(lower)alkyl" may include the same groups as mentioned for compound (I).
   The "ar(lower) alkyl" mentioned above may have one or more (preferably 1~3) suitable subs tituents typically selected from among said lower alkyl, said halogen, hydroxy, and said lower alkoxy.
   Suitable "alicyclic group" and "alicyclic moiety" of said "alicyclic(lower)alkyl" may include cyclo(C₃-C₈)alkyl, such as cyclopropyl, cyclobutyl, cyclopentyl, cyclohexyl, cycloheptyl, cyclooctyl, etc. [particularly preferred are cyclo(C₃-C₆)alkyl]; (C₇-C₁₂)bicycloalkyl or (C₇-C₁₂)bicycloalkenyl [particularly preferred are groups of the formula: 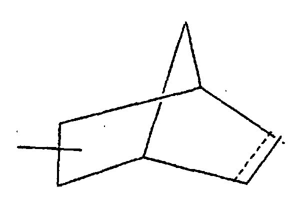 (wherein 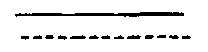 represents a single bond or a double bond), groups of the formula: 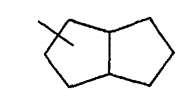 , etc.]; and (C₇-C₁₂)tricycloalkyl [particularly preferred are groups of the formula 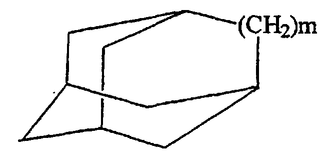 (wherein m is 0 or 1)]; among others.
   Suitable "heterocycle group" and "heterocyclic moiety" of said "heterocyclic(lower)alkyl group" may include the same groups as already mentioned for "heterocyclic group" in the description of the compound (I).
   Suitable "aryl" may include the same aryl groups as mentioned by way of example for the compound (I).
   Suitable "lower alkyl" of said "alicyclic(lower)alkyl group" and of said "heterocyclic(lower)alkyl group" may include the same groups as mentioned previously for the "lower alkyl group".
   The alicyclic group, aryl group, heterocyclic group, alicyclic(lower)alkyl group, ar (lower) alkyl group, and heterocyclic(lower)alkyl group for R¹¹ may each have one or more (preferably 1~3) suitable substituents, which are typically selected from among oxo, hydroxy, amino, said lower alkyl, carboxy, and the protected carboxy mentioned for compound (I).
   The "alicyclic group" and "aryl group" for R¹³ and R¹⁴ may each have one or more (preferably 1~3) suitable substituents typically selected from among said lower alkyl, hydroxy, the same lower alkoxy as mentioned for compound (I), said halogen, amino and nitro.
   Suitable "lower alkylene" may include methylene, ethylene, 1-methylethylene, trimethylene, tetramethylene, pentamethylene, hexamethylene, etc., with (C₁-C₄)alkylene being particularly preferred.
   The preferred exemplary groups for the xanthine compound (III) are as follows.
   R⁹ and R¹⁰ each is preferably a lower alkyl, more preferably a (C₁-C₄)alkyl, with propyl being the most preferred.
   The preferred group for R¹¹ includes cyclo (C₃-C₈) alkyl which may have oxo; (C₇-C₁₂)tricyclo-alkyl and groups of the formula: 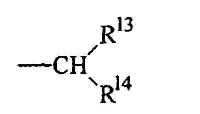 (wherein R¹³ and R¹⁴ each is a cyclo (C₃-C₈)alkyl). The more preferred are cyclo (C₃-C₆)alkyl which may have oxo; groups of the formula: 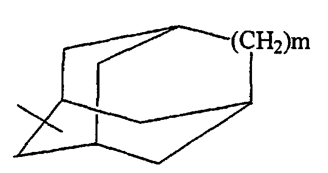 (whereas m is as defined above), and groups of the formula : 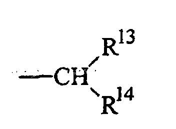 (wherein R¹³ and R¹⁴ each is cyclo(C₃-C₆)alkyl). The most preferred is cyclopentyl which may have oxo; groups of the formula: 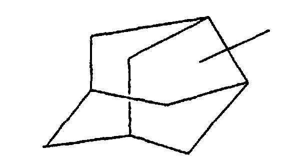 , and groups of the formula 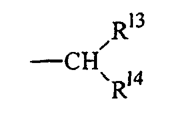 (wherein R¹³ and R¹⁴ each is cyclopropyl).
   Preferably, R¹² is hydrogen.
   Preferably, X¹ and X² each is an oxygen atom.
   The pharmaceutical composition for use in the practice of this invention can be provided and administered either in the form of the adenosine A₁A₂ₐ-receptor dual antagonist or its salt as a bulk or in a solid, semisolid or liquid form containing said adenosine A₁A₂ₐ-receptor dual antagonist or salt as an active ingredient in combination with an organic or inorganic carrier or excipient suited for rectal, oral or parenteral (inclusive of subcutaneous, intravenous and intramuscular) administration or inhalation.
   The active ingredient can be formulated with any of the nontoxic pharmaceutically acceptable carriers which are usually incorporated in various dosage forms such as tablets, pellets, troches, capsules, suppositories, aerosols, powders for inhalation, solutions, emulsions, and suspensions. Where necessary, various adjuvants, stabilizers, thickeners, coloring agents and flavoring agents can also be incorporated. The adenosine A₁A₂ₐ-receptor dual antagonist inclusive of its salt is incorporated in any such dosage form in a sufficient amount to yield the expected therapeutic benefit which depends on the stage or status of illness.
   Such pharmaceutical preparations for use in this invention can be manufactured by the established procedures in the art. Where necessary, the methods in common use in the art for improving the bioavailability of medicinal substances can also be applied to the manufacture of such pharmaceutical preparations.
   The therapeutically effective amount of the adenosine A₁A₂ₐ-receptor dual antagonist inclusive of its salt varies with the patient's age and other factors but generally the adenosine A₁A₂ₐ-receptor dual antagonist can be administered in a daily dose of 0.01~200 mg per kg human body weight.

### EFFECT OF THE INVENTION

As evidence of the usefulness of this invention, the results of concrete pharmacological tests are mentioned below.

### Test compound

In the tests mentioned below,
(A) 3-[2-(Thiazol-2-ylmethyl)-3-oxo-2,3-dihydro-pyridazin-6-yl]-2-phenylpyrazolo[1,5-a]pyridine was used as an adenosine A₁A₂ₐ-receptor dual antagonist.

### Test 1 Anticataleptic action

### Method

Mice were orally dosed with the test compound as a suspention in 0.5% of methyl cellulose and, 30 minutes later, intraperitoneally dosed with 0.32 mg/kg of haloperidol. After another 30 minutes, the animals were observed for signs of catalepsy.

### Results

| Dosage (mg/kg) | Incidence of catalepsy (%) |
|---|---|
| Control | 100 |
| 1 | 20 |

### Test 2 Anxiolytic action

### Method

Two male SD rats acclimated for 1 week and handled once were used in sets. One hour before beginning of the test, both rats in each set were orally dosed with the same amount of the test compound or the vehicle. Immediately thereafter, both rats were placed in a new environment where territories were to be established as yet and their behaviors were monitored and recorded over 15 minutes. After the test, the records were analyzed and evaluated in terms of the total duration of social interaction (social interaction time) (observation items: sniffing, following, grooming, kicking, boxing, biting, wrestling, crawling under or over the partner).

### Results

| Dosage (mg/kg) | Social interaction time (sec.) |
|---|---|
| Control | 51.3±6.0 |
| 0.32 | 90.5±12.5 |

### Test 3 Impaired-memory ameliorating action

### Method

Male Wistar rats were serially subjected to an acclimation trial, an acquisition trial 1 hour after the acclimation trial, and a retention trial 24 hours after the acquisition trial. Scopolamine 1 mg/kg was intraperitoneally administered 30 minutes before the acquisition trial. The test compound was intraperitoneally administered immediately after the acquisition trial.

### Results

| Dosage (mg/kg) | Reaction latency in retention trial (sec.) |
|---|---|
| Intact group | 300±0 |
| Scopolamine 1 mg/kg+control | 80.2±34.9 |
| Scopolamine 1 mg/kg + 1 | 215.6±35.0 |

Based on the above test results, an adenosine A₁A₂ₐ-receptor dual antagonist was found to have anticataleptic, anxiolytic and impaired-memory ameliorating actions and, therefore, considered to be of use as a drug for the prevention and/or treatment of Parkinson's disease and concomitant symptom(s) thereof such as anxiety, depression and/or memory impairment, among others.

In the above mentioned, an adenosine A₁A₂ₐ-receptor dual antagonist was explained as a single compound, however, it is possible to gain a similar effect to the single compound even if a combination drug consisting of an adenosine A₁-receptor antagonist and an adenosine A₂ₐ-receptor antagonist in a suitable combination rate.

Suitable compounds of an adenosine A₁-receptor antagonist and an adenosine A₂ₐ-receptor antagonist can be judiciously selected from various compounds above concretely mentioned.

The combination rate of the both compounds can be judiciously decided, and the affinity for the adenosine A₁-receptor is preferably 0.25~40 times, more preferably 8~40 times, as high as its affinity for the adenosine A₂ₐ receptor as a whole of the combination drug.

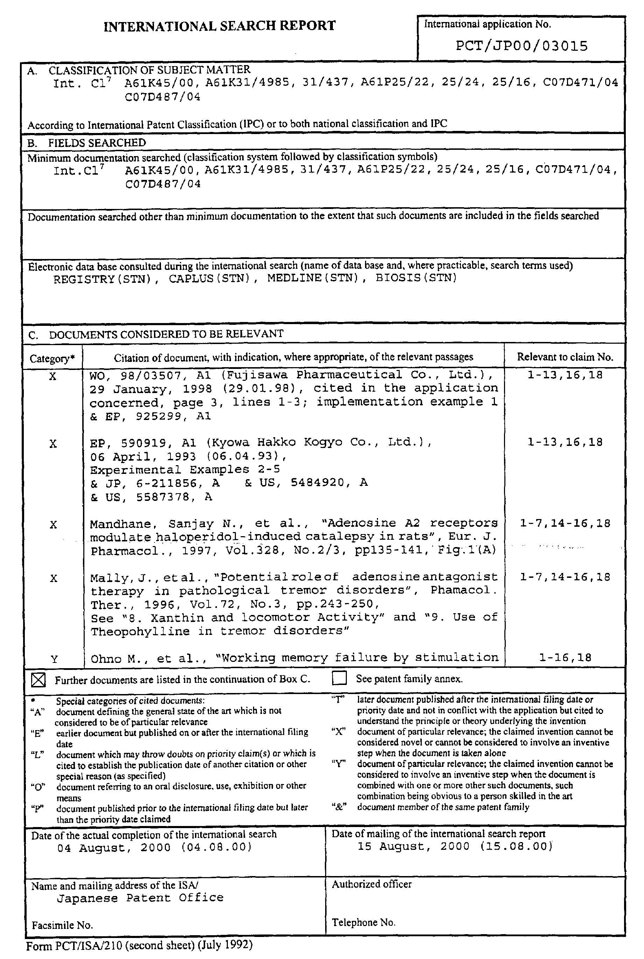

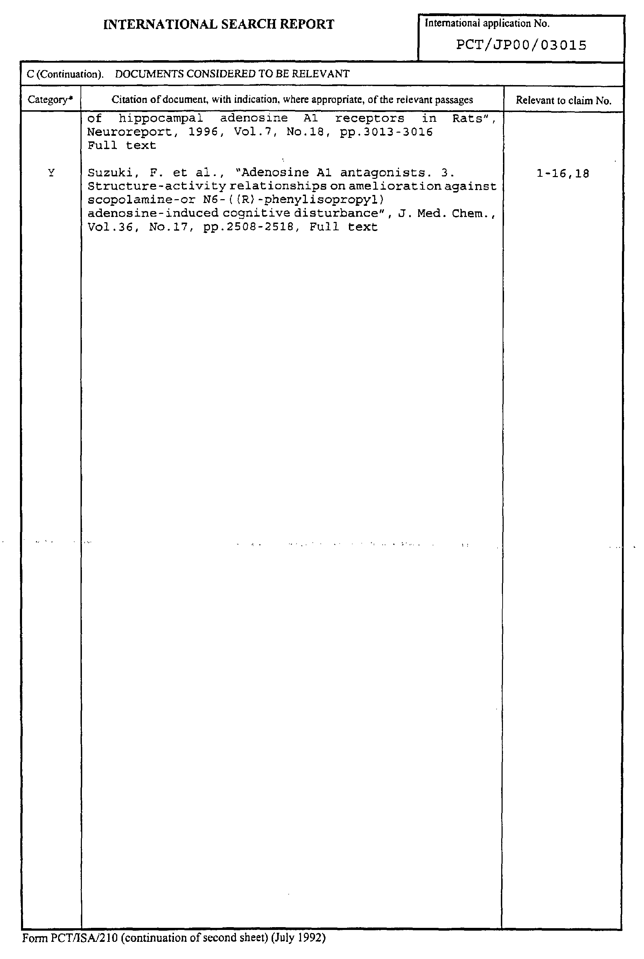

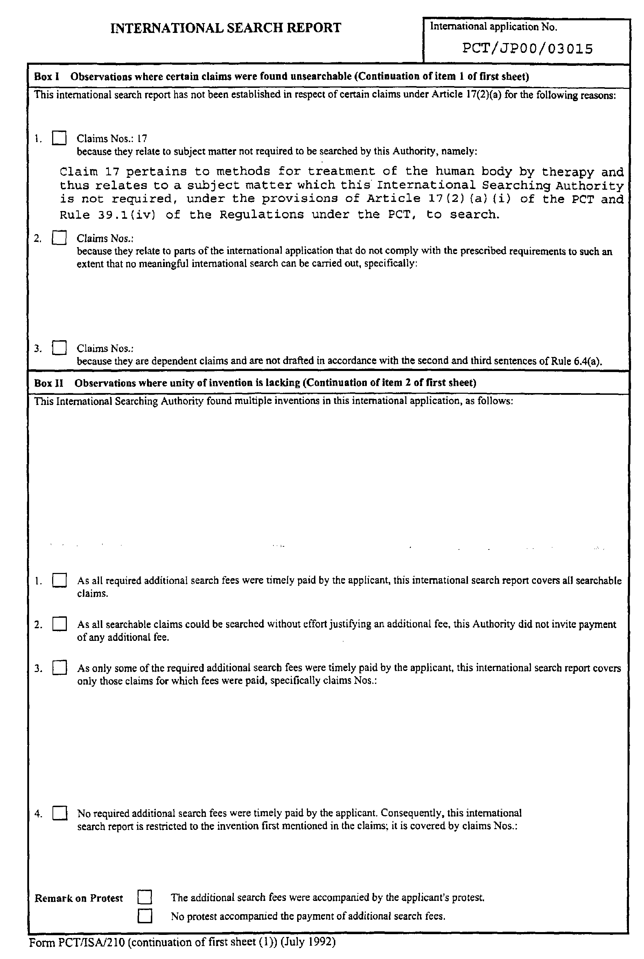

## Claims

1. A pharmaceutical composition for the prevention and/or the treatment of Parkinson's disease and the concomitant symptom(s) thereof, which comprises an adenosine A₁A₂ₐ-receptor dual antagonist or a salt thereof as an active ingredient.

2. A pharmaceutical composition claimed in Claim 1, wherein the concomitant symptom(s) is(are) anxiety, depression and/or memory impairment.

3. A pharmaceutical composition claimed in Claim 1 or 2, wherein the adenosine A₁A₂ₐ-receptor dual antagonist has an adenosine A₂ₐ-receptor antagonizing action of not more than 100 nM in terms of IC₅₀.

4. A pharmaceutical composition claimed in Claim 3, wherein the adenosine A₁A₂ₐ-receptor dual antagonist has an adenosine A₂ₐ-receptor antagonizing action of notmore than 50 nM in terms of IC₅₀.

5. A pharmaceutical composition claimed in Claim 1 to 4, wherein the affinity for the adenosine A₁-receptor of the adenosine A₁A₂ₐ-receptor dual antagonist is 0.25 to 40 times as high as that for the adenosine A₂ₐ-receptor.

6. A pharmaceutical composition claimed in Claim 5, wherein the affinity for the adenosine A₁-receptor of the adenosine A₁A₂ₐ-receptor dual antagonist is 8 to 40 times as high as that for the adenosine A₂ₐ-receptor.

7. A pharmaceutical composition claimed in Claim 1 or 2, wherein the adenosine A₁A₂ₐ-receptor dual antagonist is selected from the group consisting of adenine derivative, barbiturate derivative, benzimidazole derivative, benzo[1,2-c:5,4-c']dipyrazole derivative, benzo[b]furan derivative, benzo[g]pteridine-2,4-dione derivative, β-carboline derivative, dibenz[b,f]azepine derivative, flavone derivative, imidazo[1,2-a]pyrazine derivative, imidazo[4,5-b]pyridine derivative, imidazo[4,5-c]quinoline derivative, imidazo[4,5-e][1,4]diazepine-5,8-dione derivative, imidazo[4,5-f]quinazoline-7,9-dione derivative, imidazo[4,5-g]quinazoline-6,8-dione derivative, imidazo[1,2-a]quinoxaline derivative, imidazoline derivative, imidazotriazolopyrimidine derivative, pteridine-2,4-dione derivative, pyrazole derivative, pyrazolo[1,5-a]pyradine derivative, pyrazolo[1,5-a]pyridine derivative, pyrazolo[3,4-b]pyridine derivative, pyrazolo[3,4-d]pyrimidine derivative, pyrazolo[4,3-d]pyrimidine derivative, pyrazolo[4,3-c]quinoline derivative, pyrimidine derivative, pyrimido[4,5-b] (tetrahydro)indole derivative derivative, pyrrolo[2,3-d]pyrimidine derivative, quinazoline derivative, quinoline derivative, thiazolo[3,2-a]pyrimidine derivative, thiazolo[2,3-b]quinazoline derivative, thiazolo[4,5-d]pyrimidine-5,7-dione derivative, thiazolo[5,4-d]pyrimidine-5,7-dione derivative, thiophene derivative, triazolo[3,2-a][2,7]naphthyridine derivative, triazolopurine derivative, [1,2,4]triazolo[4,3-b]pyridazine derivative, triazolo[1,5-a]pyrimidine derivative, triazolo[1,5-c]pyrimidine derivative, [1,2,4]triazolo[1,5-c]quinazoline derivative, [1,2,4]triazolo[4,3-a]quinoxaline derivative, triazolo[1,5-a]triazine derivative, xanthine derivative, mesoionic xanthine derivative.

8. A pharmaceutical composition claimed in Claim 7, wherein the adenosine A₁A₂ₐ-receptor dual antagonist is a compound selected from the pyrazolopyridine compounds of the general formula or a salt thereof: 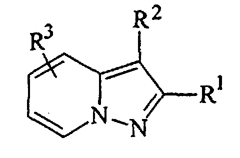 [wherein R¹ is lower alkyl, aryl optionally having one or more suitable substituent(s) or a heterocyclic group;
R² is a group of the formula: 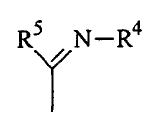 (wherein R⁴ is a protected amino or hydroxy and R⁵ is hydrogen or lower alkyl);
cyano;
a group of the formula:
-A-R⁶
(wherein R⁶ is acyl and A is lower aliphatic hydrocarbon group optionally having one or more suitable
substituent(s));
amidated carboxy;
unsaturated heterocyclic group optionally having one or more suitable substituent(s) ;
amino; or
protected amino; and
R³ is hydrogen, lower alkyl, lower alkoxy, or halogen]

9. A pharmaceutical composition claimed in Claim 8, wherein the adenosine A₁A₂ₐ-receptor dual antagonist is selected from the pyrazolopyridine compounds of the general formula: 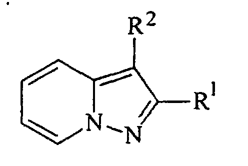 [wherein R¹ is aryl which may be substituted by halogen and R² is dihydropyridazinyl group having lower alkyl optionally substituted by an unsaturated 3~8-membered monocyclic heterocyclic group containing 1 or 2 sulfur atoms and 1~3 nitrogen atoms or acyl(lower)alkyl and oxo; dihydropyridazinyl group having cyclo(lower)alkyl substituted by acyl(lower)alkyl or
acyl(lower)alkylidene and oxo ; or dihydropyridazinyl having cyclo(lower)alkenyl substituted by
acyl(lower)alkyl or acyl(lower)alkylidene and oxo].

10. A pharmaceutical composition claimed in Claim 9, wherein the adenosine A₁A₂ₐ-receptor dual antagonist is a compound, in which
R¹ is phenyl or phenyl substituted by halogen, and
R² is 3-oxo-2,3-dihydropyridazinyl group having thiazolyl(lower)alkyl group or
3-oxo-2,3-dihydropyridazinyl group having lower alkyl.

11. A pharmaceutical composition claimed in Claim 10, wherein the adenosine A₁A₂ₐ-receptor dual antagonist is 3-[2-(thiazol-2-ylmethyl)-3-oxo-2,3-dihydro-pyridazin-6-yl]-2-phenylpyrazolo[1,5-a]pyridine.

12. A pharmaceutical composition claimed in Claim 1 or 2, wherein the adenosine A₁A₂ₐ-receptor dual antagonist is a compound selected from the pyrazolopyrazine compounds of the following general formula or a salt thereof: 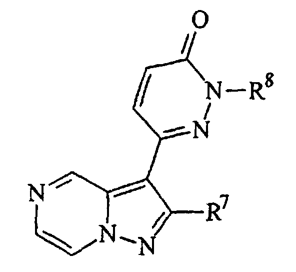 [wherein R⁷ is aryl optionally having one or more suitable substituent(s); R⁸ is hydrogen, lower alkyl, cyclo(lower)alkyl, lower alkyl substituted by cyclo(lower)alkyl, ar(lower)alkyl , a heterocyclic group, or lower alkyl substituted by heterocyclic group] and salts thereof.

13. A pharmaceutical composition claimed in Claim 12, wherein the adenosine A₁A₂ₐ-receptor dual antagonist is a compound, in which
R⁷ is phenyl or phenyl substituted by halogen, and
R⁸ is lower alkyl or a heterocyclic group.

14. A pharmaceutical composition claimed in Claim 1 or 2, wherein the adenosine A₁A₂ₐ-receptor dual antagonist is a compound selected from the xanthine compounds of the general formula or a salt thereof: 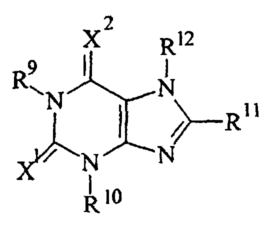 [wherein R⁹, R¹⁰ and R¹² each is hydrogen, lower aliphatic hydrocarbon group optionally having one or more suitable substituent(s), higher alkyl optionally having one or more suitable substituent(s) or ar(lower)alkyl optionally having one or more suitable substituent(s); R¹¹ is hydrogen; alicyclic, aryl, heterocyclic, alicyclic(lower)alkyl, ar(lower)alkyl or heterocyclic(lower)alkyl, each of which may have one or more suitable substituent(s); or a group of the formula: 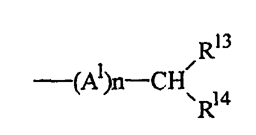 (wherein R¹³ and R¹⁴ each is an alicyclic group optionally having one or more suitable substituent(s) or aryl optionally having one or more suitable substituent(s) ; A¹ is lower alkylene; and n is 0 or 1); and X¹ and X² each is an oxygen atom or a sulfur atom] and salts thereof.

15. A pharmaceutical composition claimed in Claim 14, wherein the adenosine A₁A₂ₐ-receptor dual antagonist is a compound, in which
R⁹ and R¹⁰ each is lower alkyl,
R¹¹ is cycle(C₃-C₈)alkyl which may have oxo;
(C₇-C₁₂)tricycloalkyl; or a group of the formula: 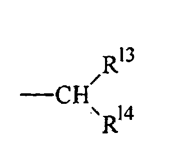 (wherein R¹³ and R¹⁴ each is cyclo(C₃-C₈)alkyl);
R¹² is hydrogen, and
X¹ and X² each is an oxygen atom.

16. Use of an adenosine A₁A₂ₐ-receptor dual antagonist for the manufacture of a pharmaceutical composition for the prevention and/or the treatment of Parkinson's disease and the concomitant symptom(s) thereof.

17. A method for the prevention and/or the treatment of Parkinson's disease and the concomitant symptom(s) thereof, which comprises administering an adenosine A₁A₂ₐ-receptor dual antagonist to a patient suffering from Parkinson's disease and the concomitant symptom(s) thereof.

18. A pharmaceutical composition for the prevention and/or the treatment of Parkinson's disease and the concomitant symptom(s) thereof, which comprises a combination of 1 or more compound(s) selected from an adenosine A₁-receptor dual antagonist and 1 or more compound(s) selected from an adenosine A₂ₐ-receptor antagonist, as active ingredients.
